# EUROPÄISCHE PATENTSCHRIFT

(11) **EP 0 450 588 B1**
(45) Veröffentlichungstag und Bekanntmachung des Hinweises auf die Patenterteilung: **28.02.1996**
(21) Anmeldenummer: 91105236.3
(22) Anmeldetag: 03.04.1991
(51) Int. Cl.: C07D 211/20, A61K 31/35

(54) **Substituierte 3-Thia- bzw. 3-Oxa-alkyl-Flavone, Verfahren zu ihrer Herstellung, Verwendung derselben, Arzneimittel auf Basis dieser Verbindungen sowie Zwischenprodukte**
Substituted 3-thia- or 3-oxa-alkyl flavones, process for their preparation, their application and drugs based on these compounds as well as intermediates
3-thia-ou 3-oxa-alkyl flavones, procédé pour leur préparation, utilisation, médicaments à la base de ces composés, aussi bien que produits intermédiaires

(30) Priorität: 06.04.1990 DE 4011187
(43) Veröffentlichungstag der Anmeldung: 09.10.1991
(73) Patentinhaber: HOECHST AKTIENGESELLSCHAFT, D-65926 Frankfurt am Main (DE)
(72) Erfinder: Beck, Gerhard, Dr., W-6000 Frankfurt am Main (DE); Schacht, Ulrich, Dr., W-6238 Hofheim am Taunus (DE); Kesseler, Kurt, Dr., W-6232 Bad Soden am Taunus (DE); Granzer, Ernold, Dr., W-6233 Kelkheim/Taunus (DE)

(56) Entgegenhaltungen:
- EP-A- 0 121 489
- EP-A- 0 122 053
- EP-A- 0 183 169
- FR-M- 6 009

## Beschreibung

Epidemiologische Studien in vielen Ländern während der letzten drei Jahrzehnte deuten auf eine Abhängigkeit zwischen erhöhten Cholesterin-Konzentrationen im Blut und dem Herzinfarktrisiko hin (z. B. The Framingham Study, Ann. Intern. Med. 74 (1971) 1-12). Insbesondere erhöhte Low Density Lipoprotein (LDL)-Plasmaspiegel werden dafür verantwortlich gemacht. Untersuchungen der letzten Jahre (W. Palinski et al., Proc. Natl. Acad. Sci. USA 86, 1372 (1989), ibid. 84, 2995 (1987) u. Kita et al., ibid 84, 5928 (1987)) haben gezeigt, daß die oxidative Modifikation der LDL-Partikel durch freie Radikale wie z. B. ^{·}OH, O₂⁻^{·} oder durch Singulett Sauerstoff für deren atherogene Wirkung verantwortlich ist. Die Oxidation der LDL wird eingeleitet durch die Oxidation der mehrfach ungesättigten Fettsäuren im LDL-Partikel. Die Abbauprodukte der Lipidperoxidation sind reaktive Aldehyde, wie Z. B. Nonenal oder Malondialdehyd, welche mit Lysinresten des LDL-Bindungsproteins Apo B reagieren (Esterbauer et al., J. Lipid. Res. 28, 495 (1987). Die chemisch veränderten LDL-Partikel werden dann über sog. Scavenger-Rezeptoren unkontrolliert von Macrophagen aufgenommen und verwandeln diese in Schaumzellen, welche sich in arteriosklerotischen Läsionen manifestieren. Die von der vorliegenden Anmeldung umfaßten Verbindungen sind LDL-spezifische Antioxidantien, welche die Oxidation der LDL-Partikel verhindern. Die Spezifität der Verbindungen wird erreicht durch Verknüpfung von antioxidativ wirksamen Flavonen mit verzweigten oder unverzweigten lipophilen Resten, die bevorzugt in LDL-Partikel inkorporieren (vgl. M. Krieger, M. J. MC Phaul, J.L. Goldstein, M.S. Brown, J. Biol. Chem 254, 3845 (1979).

Flavone, die strukturell den Flavonen der vorliegenden Anmeldung ähneln, wurden in EP-A-0 121 489 beschrieben.

Gegenstand der vorliegenden Erfindung sind Flavone der Formel I, 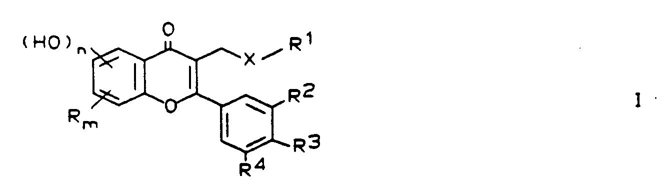
in der bedeuten:
- X: Schwefel oder Sauerstoff
- R: Halogen, (C₁-C₄)-Alkyl oder Trifluor-methyl,
- m: null, 1, 2 oder 3,
- n: 1 oder, falls m = null, 1 oder 2 ist, auch 2, R¹ (C₃-C₂₅)-Alkenyl, wobei gegebenenfalls eine CH₂-Gruppe durch Sauerstoff ersetst ist, oder (C₃-C₂₅)-Alkenyl, das mit Cyclohexenyl, das seinerseits 1 - 3 Methylgruppen enthält, substituiert ist,
- R²,R³,R⁴: Wasserstoff, Hydroxy, Halogen, (C₁-C₄)-Alkyl, (C₁-C₄)-Alkoxy, Trifluormethyl, wobei R², R³, R⁴ gleich oder verschieden sind.

Falls m 2 oder 3 ist, sind die Reste R gleich oder verschieden.

In den vorstehenden und folgenden Ausführungen bedeuten Alkyl, Alkenyl und Alkoxy stets geradkettige oder verzweigte Reste. Halogen steht für Fluor, Chlor oder Brom.

Sofern die Reste R¹ ein oder mehrere Asymmetriezentren enthalten, sind die reinen Enantiomeren, Diastereomeren und Racemate umfaßt. Ebenfalls beansprucht werden für R¹ = (C₃-C₂₅)-Alkenyl die möglichen E- bzw. Z-Isomere. Die Alkenylreste sind vorzugsweise 1 bis 6-fach ungesättigt.

Unter den Substituenten sind bevorzugt:
- X: Schwefel oder Sauerstoff
- R: Fluor, Chlor, (C₁-C₃)-Alkyl oder Trifluormethyl,
- m: null, 1, 2 oder 3,
- n: 1 oder, falls m null, 1 oder 2 ist, auch 2,
- R¹: (C₄-C₂₂)-Alkenyl, wobei gegebenenfalls eine CH₂-Gruppe durch Sauerstoff ersetzt sein kann,
- R²,R³,R⁴: Wasserstoff, Hydroxy, Fluor, Chlor, (C₁-C₃)-Alkyl, (C₁-C₃)-Alkoxy, Trifluormethyl, wobei R², R³, R⁴ gleich oder verschieden sind.
Unter den Substituenten sind besonders bevorzugt:
- X: Schwefel
- R: Fluor, Chlor, Methyl oder Trifluormethyl,
- m: null, 1, 2 oder 3,
- n: 1 oder, falls m null, 1 oder 2 ist, auch 2,
- R¹: (C₄-C₂₂)-Alkenyl, wobei gegebenenfalls eine CH₂-Gruppe durch Sauerstoff ersetzt sein kann,
- R²,R³,R⁴: Wasserstoff, Hydroxy, Fluor, Chlor, Methyl, Methoxy, Trifluormethyl, wobei R², R³, R⁴ gleich oder verschieden sind.

Gegenstand der Erfindung ist ferner ein Verfahren zur Herstellung der Verbindungen der Formel I, das dadurch gekennzeichnet ist, daß man
a) ein Phenol der Formel II 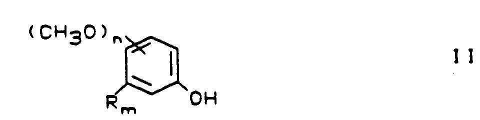 worin m, n und R die zur Formel I angegebene Bedeutung haben,
   durch Acylierung in die Verbindungen der Formel III überführt 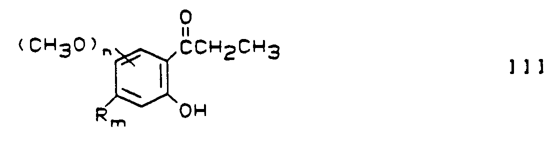 worin m, n und R die zur Formel I angegebene Bedeutung haben,
b) eine Verbindung der Formel III an der freien phenolischen Hydroxylgruppe mit einem Säurehalogenid der Formel IV, 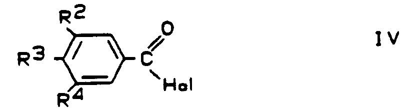 worin R², R³, R⁴ die zur Formel I angegebene Bedeutung haben und Hal gleich Brom oder Chlor ist, acyliert zu einer Verbindung der Formel V 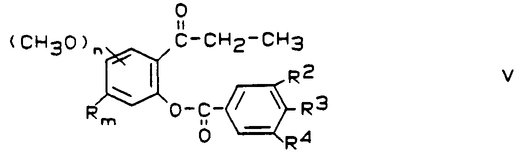 worin m, n, R und R², R³, R⁴ die zur Formel I angegebene Bedeutung haben,
c) eine Verbindung der Formel V cyclisiert zu einer Verbindung der Formel VI 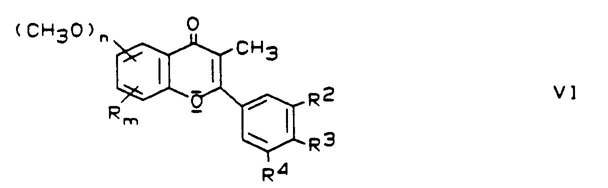 worin m, n, R und R², R³, R⁴ die zur Formel I angegebene Bedeutung haben,
d) in einer Verbindung der Formel VI die Methoxygruppen abspaltet, zu einer Verbindung der Formel VII 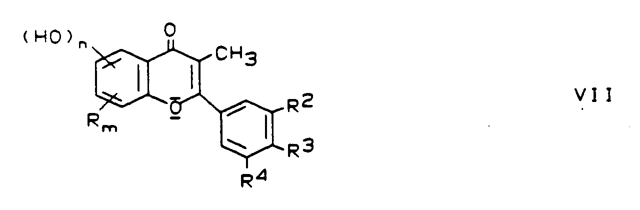 worin m, n und R sowie R², R³, R⁴ die zur Formel I angegebene Bedeutung haben,
e) eine Verbindung der Formel VII mit einem Acylierungsmittel zu einer Verbindung der Formel VIII umsetzt, 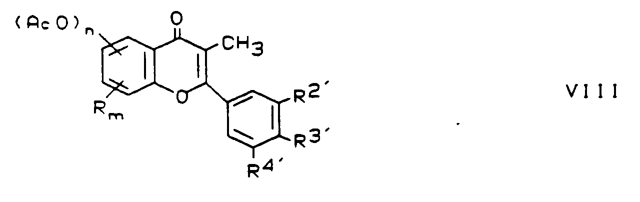 worin m, n und R die zur Formel I angegebene Bedeutung haben, und R^{2'}, R^{3'}, R^{4'} Wasserstoff, Halogen, (C₁-C₄)-Alkyl, (C₁-C₄)-Alkoxy, Trifluormethyl oder Acyloxy sind, wobei R^{2'}, R^{3'}, R^{4'} gleich oder verschieden sind,
f) eine Verbindung der Formel VIII selektiv halogeniert zu einer Verbindung der Formel IX 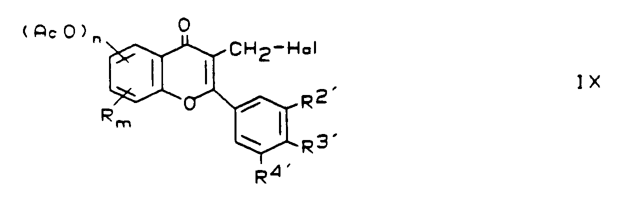 worin m, n und R die zur Formel I, R^{2'}, R^{3'}, R^{4'} die zur Formel VIII angegebene Bedeutung haben und Hal gleich Chlor, Brom oder Jod ist,
g) eine Verbindung der Formel IX umsetzt mit einer Verbindung der Formel X

   H-X-R¹ X

   worin X und R¹ die zur Formel I angegebene Bedeutung haben,
   zu einer Verbindung der Formel XI 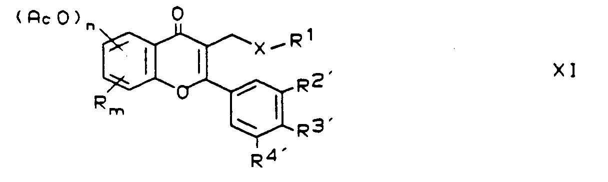 worin m, n, X, R und R¹ die zur Formel I und R^{2'}, R^{3'}, R^{4'} die zur Formel VIII angegebene Bedeutung haben,
h) in einer Verbindung der allgemeinen Formel XI die Acylschutzgruppen abspaltet zu einer Verbindung der allgemeinen Formel I.

Die im erfindungsgemäßen Verfahren als Ausgangsmaterialien verwandten Phenole der allgemeinen Formel II sind kommerziell erhältlich (z. B. Fluka, Aldrich) bzw. nach literaturbekannten Verfahren (z. B. R.O. Duthaler, Helv. Chim. Acta 67, 1411 (1984) oder M.V. Sargent et al., J. Chem. Soc. 1974, S. 1353)) herstellbar.

Zur Herstellung der substituierten Propiophenone III werden die Phenole der Formel II nach Friedel-Crafts z. B. mit Propionsäurechlorid in Gegenwart von AlCl₃ oder BF₃-Ätherat acyliert, eine bevorzugte Ausführungsform besteht jedoch darin, analog dem Verfahren zu arbeiten, welches von Canter, F. W. et al., J. Chem. Soc. 1 (1931) 1245 beschrieben wurde. Die anschließende O-Acylierung der Propiophenone III erfolgt mit den Säurehalogeniden der Formel IV zweckmäßigerweise in einem Lösungsmittel wie Tetrahydrofuran, Aceton, DMF, Dimethylsulfoxid in Gegenwart einer geeigneten Base wie z. B. Triethylamin, K₂CO₃, Natriumhydrid, Kalium-tert.-butylat, Natriumethylat oder Butyllithium bei Temperaturen von -2O°C bis +5O°C über 1 - 6 Stunden.

Die Säurechloride IV sind kommerziell erhältlich oder nach literaturbekannten Verfahren herstellbar (Organikum, Verlag VEB, S. 387-88, 4. Auflage 1964, Berlin).

Die O-acylierten Propiophenone der Formel V werden z. B. in einem Lösungsmittel wie Dimethylformamid oder Dimethylsulfoxid in Gegenwart von Natriumhydrid oder Na-Ethylat bei Temperaturen von -25°C bis +8O°C während 1 - 8 Stunden cyclisiert.

In den cyclisierten Verbindungen der Formel VI werden die Methoxyschutzgruppen durch Ätherspaltung entfernt. Dies kann z. B. durch Behandlung in Methylenchlorid oder Chlorform in Gegenwart von Bortribromid bei -10°C bis +30°C über 1 - 4 Stunden erfolgen. Durch Erwärmen mit Jodwasserstoffsäure (48 %ig) oder konzentrierter Bromwasserstoffsäure (48 %ig) lassen sich die Methoxygruppen ebenfalls abspalten. Dabei werden im allgemeinen bei -10°C bis +25°C nur die im Benzopyronrest vorhandenen Methoxygruppen abgespaltet, während für die Abspaltung von Methoxygruppen (z. B. R², R³ oder R⁴) im 2-ständigen Phenylrest Temperaturen von +40°C bis +110°C nötig sind.

Die freien Hydroxylgruppen in den Verbindungen der Formel VII werden durch Acylierung geschützt. Als Acylierungsmittel eignen sich aliphatische oder aromatische Säurehalogenide oder Säureanhydride. Die Acylierung wird vorzugsweise in Lösungsmitteln wie Methylenchlorid, Chloroform, Toluol bei Temperaturen zwischen 0°C und +40°C in Gegenwart von Basen, wie z. B. Triethylamin, Pyridin etc. durchgeführt. Die bevorzugte Ausführungsform besteht jedoch in der Acylierung in Pyridin mit Essigsäureanhydrid bei Temperaturen von +30°C bis +90°C innerhalb 1/2 - 2 Stunden.

Die acylierten Verbindungen der Formel VIII werden an der 3-CH₃-Gruppe selektiv halogeniert. Als Halogenierungsmittel können dabei N-Hal-succinimide (Hal = Cl, Br, J) verwendet werden oder elementares Chlor oder Brom in Gegenwart von Radikalbildnern wie z. B. Azoisobutyronitril oder Benzoylperoxid oder unter Bestrahlen mit UV- Licht. Besonders einfach gelingt die Bromierung durch Erwärmen der Verbindungen VIII in Tetrachlorkohlenstoff in Gegenwart von N-Bromsuccinimid bei 0°C - +50°C unter gleichzeitiger Bestrahlung mit UV-Licht.

Die Umsetzung der Halogenide der Formel IX mit den Alkoholen bzw. Mercaptanen der Formel X zu den Verbindungen der Formel XI wird z. B. bei -30°C bis +60°C in Lösungsmitteln wie Tetrahydrofuran, DME, Diäthyläther, DMF, Dimethylsulfoxid in Gegenwart von 1 eq. Base wie z. B. Natriumhydrid, Buthyllithium, Triäthylamin, Natriumäthylat durchgeführt. Die geradkettigen Alkohole der Formel X (R¹-OH) sind kommerziell erhältlich oder können nach allgemein bekannten Methoden hergestellt werden (z. B. Organikum, Verlag VEB Berlin 1964, Methodenregister S. 4, Alkohole). Die verzweigten Alkohole wie z. B. Geraniol, Nerol, Farnesol und Phytol sind ebenfalls kommerziell erhältlich (Aldrich Chemie, Steinheim) oder werden wie von J.W.K. Burrell, J. Chem. Soc. (C) 1966, S. 2144 - 2154 beschrieben, hergestellt. Die Mercaptane der Formel X (R¹S-H) werden aus den entsprechenden Alkoholen hergestellt durch Überführung in die Bromide mit Phosphortribromid (z. B. O. Isler et al., Helv. Chim. Acta 108, S. 903 (1956)) und Umsetzung der Bromide mit Thioharnstoff mit anschließender alkalischer Hydrolyse der S-Alkylthiuroniumsalze, analog wie z. B. im Organikum, Verlag VEB, Berlin 1964, S. 176 beschrieben, hergestellt. Die Mercaptane können auch direkt, wie von R.P. Valante in Tetrahedron Letters 22, 3119 (1981) beschrieben, aus den entsprechenden Alkoholen hergestellt werden.

Zur Herstellung der erfindungsgemäßen Verbindungen der Formel I werden die Acylschutzgruppen in den Verbindungen der Formel XI abgespalten. Dazu werden die Verbindungen XI vorzugsweise in Lösungsmitteln, wie z. B. Alkoholen, DME/H₂O oder dipolaren aprotischen Lösungsmitteln wie DMF, Acetonitril und DMSO gelöst und mit mehreren Äquivalenten K₂CO₃ oder NaOH versetzt und bei Temperaturen von 0°C bis +40°C 1 - 2 Stunden verseift. Eine bevorzugte Ausführungsform besteht im Lösen in Methanol oder Äthanol und Behandlung mit 2 - 4 Äquivalenten Kaliumcarbonat bei Zimmertemperatur über 2 Stunden.

Sofern die einzelnen Reaktionsprodukte nicht bereits in genügend reiner Form anfallen, so daß sie für den folgenden Reaktionsschritt eingesetzt werden können, empfiehlt sich eine Reinigung mittels Kristallisation, Säulen-, Dünnschicht- oder Hochdruckflüssigkeitschromatographie.

Außer den in den Beispielen beschriebenen Verbindungen lassen sich nach dem erfindungsgemäßen Verfahren die folgenden Verbindungen herstellen:
2-(4',5'-Dihydroxyphenyl)-3-(2-thia-octadec-1-yl)-5,7-dihydroxy-4-H-benzopyran-4-on
2-(4',5'-Dihydroxyphenyl)-3-(2-thia-5,9R,13R,17-tetramethyl-4E-octadecen-1-yl)-5,7-dihydroxy-4-H-benzopyran-4-on
2-(4',5'-Dihydroxyphenyl)-3-(2-thia-5,9,13-trimethyl-4E,8E,12E-tetradecatrien-1-yl)-5,7-dihydroxy-4-H-benzopyran-4-on
2-(4',5'-Dihydroxyphenyl)-3-(2-thia-10-oxa-5,9,9-trimethyl-4Z-dodecen-1-yl)-5,7-dihydroxy-4-H-benzopyran-4-on
2-(4',5'-Dihydroxyphenyl)-3-(2-thia-5,9-dimethyl-4E,8E-decen-1-yl)-5,7-dihydroxy-4-H-benzopyran-4-on
2-(4',5'-Dihydroxyphenyl)-3-(2-thia-5,9-dimethyl-11-(2,6,6-trimethyl-1-cyclohexenyl)-4E,6E,8E,1OE-undecen-1-yl)-5,7-dihydroxy-4-H-benzopyran-4-on
2-(4'-Chlorphenyl)-3-(2-thia-octadec-1-yl)-6-hydroxy-4-H-benzopyran-4-on
2-(4'-Fluorphenyl)-3-(2-thia-5,9R,13R,17-tetramethyl-4E-octadecen-1-yl)-6,8-dihydroxy-4-H-1-benzopyran-4-on
2-(4'-Fluorphenyl)-3-(2-thia-5,9R,13R,17-tetramethyl-4E-octadecen-1-yl)-6-hydroxy-4H-1-benzopyran-4-on
2-(4',5'-Dihydroxyphenyl)-3-(2-thia-5,9R,13R,17-tetramethyl-4E-octadecen-1-yl)-6,8-dihydroxy-4H-benzopyran-4-on
2-(4'-Fluorphenyl)-3-(2-thia-5,9R,13R,17-tetramethyl-4E-octadecen-1-yl)-7,8-dihydroxy-4H-benzopyran-4-on
2-(4'-Methoxyphenyl)-3-(2-thia-5,9R,13R,17-tetramethyl-4E-octadecen-1-yl)-6,8-dihydroxy-4H-benzopyran-4-on
2-(4'-Hydroxyphenyl)-3-(2-thia-5,9-dimethyl-11-(2,6,6-trimethyl-1-cyclohexenyl)-4E,6E,8E,1OE-undecen-1-yl)-5,7-dihydroxy-4H-benzopyran-4-on
2-(4'-Methoxyphenyl)-3-(2-thia-5,9,13-trimethy-4E,8E,12E-tetradecatrien-1-yl)-5,7-dihydroxy-4H-benzopyran-4-on
2-(4'-Methoxyphenyl)-3-(2-thia-octadec-1-yl)-5,7-dihydroxy-4-benzopyran-4-on
2-(4'-Chlorphenyl)-3-(2-thia-5,9R,13R,17-tetramethyl-4E-octadecen-1-yl)-6,8-dihydroxy-4H-benzopyran-4-on
2-(4',5'-Dihydroxyphenyl)-3-(2-thia-5,9R,13R,17-tetramethyl-4E-octadecen-1-yl)-7,8-dihydroxy-4H-benzopyran-4-on
2-(4'-Fluorphenyl)-3-(2-oxa-5,9R,13R,17-tetramethyl-4E-octadecen-1-yl)-5,6-dihydroxy-4H-benzopyran-4-on
2-(4'-Fluorphenyl)-3-(2-oxa-5,9-dimethyl-11-(2,6,6-trimethyl-1-cyclohexenyl)-4E,6E,8E,1OE-undecen-1-yl)-5,7-dihydroxy-4H-benzopyran-4-on
2-(4 '-Fluorphenyl)-3-(2-oxa-octadec-1-yl)-5,7-dihydroxy-4H-benzopyran-4-on

### Biologische Testsysteme:

### 1. Hemmung der LDL-Oxidation

Die Methode zur Prüfung der antioxidativen Wirkung der Substanzen basiert auf der Hemmung der Cu²⁺-katalysierten LDL-Oxidation durch wirksame Verbindungen, wobei als Maß für die LDL-Oxidation der Fluoreszenzanstieg bei 430 nm dient (U.P. Steinbrecher, J. Biol. Chem. 262. 3603 (1987)).

Für die LDL-Isolierung verwendete man Blut frisch geschlachteter Schweine, das in Gegenwart von EDTA (1 mg/ml) und NaN₃ (0,1 mg/ml) gesammelt und dann zur Entfernung der zellulären Bestandteile 20 min bei 2.000 x g und 4°C zentrifugiert wurde. Schrittweise Ultrazentrifugation des Plasmas in NaCl/NaBr-Lösungen der Dichten d = 1,019 und d = 1,063 lieferte die LDL-Fraktion (R.J. Havel, H.A. Eder und J.H. Bragdon, J. Clin. Investig. 34, 1345 (1955)). Die Proben wurden hierbei im Festwinkelrotor 50.2 Ti (Beckman Instruments) jeweils 18 Stunden bei 300.000 x g und 17°C zentrifugiert. Zur Verminderung der EDTA-Konzentration mußte die so gewonnene LDL-Fraktion während 48 Stunden einer zweimaligen Dialyse im 100fachen Volumen einer phosphatgepufferten Kochsalzlösung (160 mM NaCl,10 mM NaH₂PO₄) pH 7,4 unterworfen werden (U.P. Steinbrecher, J.L. Witztum, S. Parthasarathy und D. Steinberg, Arteriosclerosis 7, 135 (1987)). Die anschließende Reinheitsprüfung der LDL-Fraktion durch Agarosegel-Elektrophorese (U.P. Steinbrecher, J.L. Witztum, S. Parthasarathy und D. Steinberg, Arteriosclerosis 7, 135 (1987)) zeigte eine einzige Bande.

Für die Oxidationsversuche verdünnte man die bei 4°C gelagerte LDL-Fraktion mit dem Dialysepuffer der obengenannten Zusammensetzung auf 0,1 mg Protein/ml. 2,5 ml dieser LDL-Lösung wurden mit 25 µl ethanolischer Lösung verschiedener Konzentrationen der Prüfsubstanz versetzt und in verschlossenen Gefäßen unter Stickstoff 1 Stunde bei 37°C inkubiert, um zunächst einen ausreichenden Einbau der Substanz in die LDL-Partikel zu erzielen (L.R. McLean und K.A. Hagaman, Biochemistry 28, 321 (1989)). Die maximale Konzentration der Prüfverbindungen betrug 1O µM im Test.

Durch Zugeben von 12,5 µl einer 1 mM CuSO₄-Lösung, d. h. 5 µM Cu²⁺ im Test, und zweistündige Inkubation bei 37°C in Zellkulturschälchen unter Luftzutritt erfolgte die Oxidation des LDL. Die Kontrollen enthielten 25 µl reines Ethanol. Zur Ermittlung des Leerwertes wurden analog vorbereitete LDL-Proben mit 12,5 µl einer Lösung aus 1 mM CuSO₄ und 40 mM EDTA versetzt und ebenfalls 2 Stunden bei 37°C inkubiert.

Nach Beendigung der Inkubation bestimmte man in allen Proben mit der Anregungswellenlänge 365 nm die emittierte Fluoreszenzintensität bei 430 nm (Spektrofluorimeter Typ LS-3 der Firma Perkin-Elmer). Für den Vergleich der Wirkungsstärke der Antioxidantien wurden die IC₅₀-Werte ermittelt, d. h. diejenigen molaren Konzentrationen, die unter den beschriebenen Testbedingungen die Fluoreszenzzunahme um 50 % hemmen (Kontrollen = 100). Hierzu wurde die Fluoreszenzmessung mit jeweils 5 bis 6 verschiedenen Konzentrationen der Prüfsubstanz als Doppelbestimmung durchgeführt. Auftragung der prozentualen Hemmwerte auf semilogarithmischem Papier und graphische Interpolation lieferte die entsprechende IC₅₀(Tabelle I). Als Vergleichsverbindung diente Vitamin E.

**Tabelle I**

| Verbindung gem. Bsp. | IC₅₀mol/Liter |
|---|---|
| 9b | 1.2 x 10⁻⁵ |
| 9d | 8.0 x 10⁻⁶ |
| 9e | 3.3 x 10⁻⁷ |
| 9i | 9.0 x 10⁻⁷ |
| Vitamin E | 4.8 x 10⁻⁶ |

### 2. Supprimierung bzw. Inhibierung der Cholesterin-biosynthese in Zellkulturen von HEP-G2-Zellen

Monolayers von HEP-G2-Zellen in lipoproteinfreiem Nährmedium wurden mit entsprechenden Konzentrationen der Prüfsubstanzen eine bestimmte Zeit (z. B. 1 Stunde) vorinkubiert, nach Zugabe des markierten Präkursors, z. B. ¹⁴C-Natriumacetat, wurde die Inkubation fortgesetzt (z. B. 3 Stunden). Nach Zugabe eines internen Standards (³H-Cholesterin) wurde ein Teil der Zellen alkalisch verseift. Die Lipide der verseiften Zellen wurden mit Chloroform/Methanol extrahiert. Dieses Lipidgemisch wurde nach Zusatz von Träger-Cholesterin präparativ dünnschichtchromatographisch getrennt, die Cholesterinbande nach dem Sichtbarmachen mit Jod-Dampf isoliert und die aus dem ¹⁴C-Präkursor gebildete Menge ¹⁴-C-Cholesterin szintigraphisch bestimmt. In einem aliquoten Teil der Zellen wurde Zellprotein bestimmt, so daß die in der Zeiteinheit pro mg Zellprotein gebildete Menge ¹⁴C-Cholesterin berechnet werden kann. Durch Vergleich dieses Wertes mit der Menge an ¹⁴C-Cholesterin, die pro mg Zellprotein und Zelteinheit einer gleichbehandelten, jedoch prüfsubstanzfreien Kultur gebildet wurde, ergab sich die Hemmwirkung des jeweiligen Prüfpräparates auf die Cholesterin-Biosynthese von HEP-G2-Zellkulturen.

**Prüfung von Substanzen auf Hemmung der Cholesterin-Biosynthese in konfluenten Zellkulturen (Monolayer) von HEP-G2-Zellen** 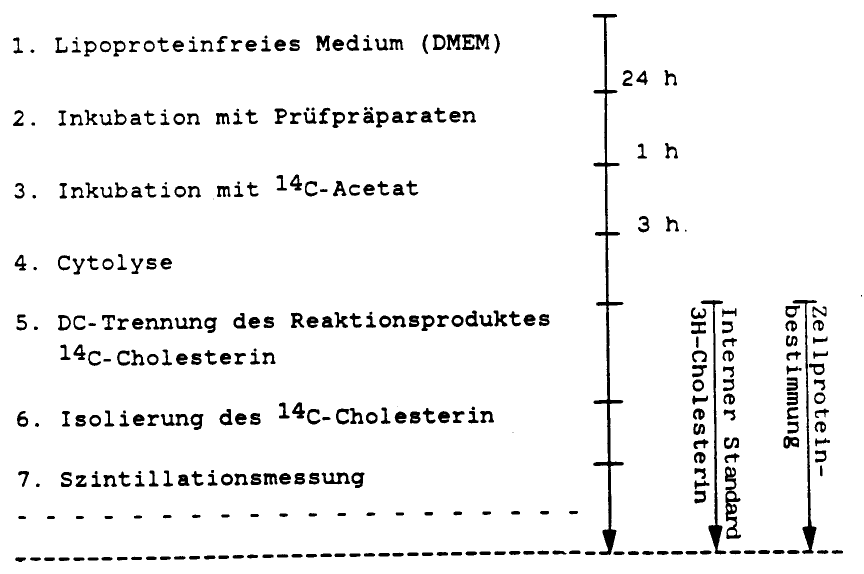 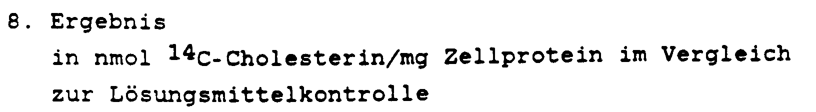
Nach der oben beschriebenen Methode wurden von den erfindungsgemäßen Verbindungen z. B. folgende Hemmwerte der Cholesterinbiosynthese (in HEP-G2-Zellen) ermittelt (der IC₅₀-Wert in Mol/Liter ist diejenige Konzentration der Verbindung, die eine 50 %ige Hemmung der Cholesterinbiosynthese bewirkt) (Tab.II).

**Tabelle II**

| Verbindung gem. Bsp. | IC₅₀ mol/Liter |
|---|---|
| 9e | 1.0 x 10⁻⁸ |
| 9i | 9.0 x 10⁻⁹ |
| 9l | 8.0 x 10⁻⁸ |
| 9m | 8.0 x 10⁻⁸ |

Die Verbindungen der Formel I zeichnen sich durch starke Hemmung der LDL-Oxidation und der Cholesterinbiosynthese aus.

Die erfindungsgemäßen Verbindungen der Formel I sind stark wirksame Antioxidantien, welche die LDL-Oxidation verhindern. Auf Grund ihrer Spezifität wirken sie stärker als endogene Antioxidantien wie z. B. Vitamin E. Die Verbindungen eignen sich daher, wie eingangs ausgeführt, zur Prophylaxe und Behandlung arteriosklerotischer Veränderungen, wie z. B. der koronaren Herzerkrankung. Sie eignen sich auch zur Behandlung anderer Krankheiten, bei denen freie Radikale maßgeblich beteiligt sind, wie z. B. Entzündungen, Rheuma, Cerebralinfarkt, Leberzirrhose, Autoimmunkrankheiten, Kataraktbildung.

Die Verbindungen der Formel I sind darüber hinaus Hemmer der Cholesterinbiosynthese und tragen durch diese Eigenschaft zu einer weiteren Senkung des arteriosklerotischen Risikos bei.

Hohe Cholesterinspiegel werden mit einer Reihe von Erkrankungen, wie z. B. der koronaren Herzerkrankung oder der Arteriosklerose, in Verbindung gebracht. Daher ist auch die Senkung erhöhter Cholesterinspiegel zur Vorbeugung und Behandlung solcher Erkrankungen ein therapeutisches Ziel.

Die Verbindungen der Formel I eignen sich daher als Antioxidantien und Hypolipidemika zur Behandlung bzw. Prophylaxe arteriosklerotischer Veränderungen.

Die Erfindung betrifft daher auch pharmazeutische Präparate auf Basis dieser Verbindungen sowie ihre Verwendung als Arzneimittel, insbesondere als Antioxidantien und Hypolipidemika und zur Prophylaxe arteriosklerotischer Veränderungen.

Die Anwendung der Verbindungen der Formel I als Antiarteriosklerotika erfolgt z. B. in oralen Dosen von 3 bis 2.500 mg pro Tag, vorzugsweise jedoch im Dosisbereich von 10 bis 500 mg. Diese Tagesdosen können nach Bedarf auch in zwei bis vier Einzeldosen aufgeteilt werden oder in Retardform verabreicht werden. Das Dosierungsschema kann vom Typ, Alter, Gewicht, Geschlecht und medizinischem Zustand des Patienten abhängen.

Ein zusätzlicher cholesterinsenkender Effekt läßt sich durch gleichzeitige Gabe der erfindungsgemäßen Verbindungen mit gallensäurebindenden Stoffen, wie z. B. Anionenaustauscherharzen, erzielen. Die Gallensäureausscheidung führt zu einer verstärkten Neusynthese und damit zu einem erhöhten Cholesterinabbau (vgl. M. S. Brown, P. T. Koranen und J. C. Goldstein, Science 212, 628 (1981); M. S. Brown , J. C. Goldstein, Spektrum der Wissenschaft 1985, 1, 96).

Die erfindungsgemäßen Verbindungen der Formel I können gelöst oder suspendiert in pharmakologisch unbedenklichen organischen Lösungsmitteln, wie ein- oder mehrwertigen Alkoholen, wie z. B. Ethanol, Ethylenglykol oder Glycerin, in Triacetin, in Alkohol-Acetaldehyddiacetalgemischen, Ölen, wie z. B. Sonnenblumenöl oder Lebertran, Ethern, wie z. B. Diethylenglykoldimethylether, oder auch Polyethern, wie z. B. Polyethylenglykol, oder auch in Gegenwart anderer pharmakologisch unbedenklicher Polymerträger wie z. B. Polyvinylpyrrollidon, oder in festen Zubereitungen zur Anwendung gelangen.

Für die Verbindungen der Formel I sind feste, oral verabreichbare Zubereitungsformen bevorzugt, die die üblichen Hilfsstoffe enthalten können. Sie werden nach üblichen Methoden hergestellt.

Als Zubereitung für die orale Anwendung eignen sich insbesondere Tabletten, Dragees oder Kapseln. Eine Dosierungseinheit enthält vorzugsweise 10 bis 500 mg Wirkstoff.

Die Verbindungen der Formeln VIII, IX und XI sind neu und stellen wertvolle Zwischenprodukte für die Herstellung von Verbindungen der Formel I dar. Die Erfindung betrifft daher auch diese Verbindungen sowie Verfahren zu ihrer Herstellung.

Vorbemerkung: NMR-Spektren wurden, sofern nichts anderes angegeben, in CDCl₃ mit internem Standard TMS gemessen. Zur Klassifizierung von NMR-Signalen gelten folgende
- Abkürzungen:: s = Singulett, d = Dublett, p = Pentett, t = Triplett, q = Quartett.

Schmelzpunkte sind unkorrigiert.

Allgemeine Vorschrift zur Herstellung von Verbindungen der allgemeinen Formel I

### Beispiel 1a (m = null, n = 2)

### 2-Hydroxy-4,6-dimethoxypropiopbenon (IIIa)

In eine Mischung von 22,7 g Zinkchlorid und 155 g (1,0 Mol) 3,5-Dimethoxyphenol (IIa) (vgl. Tab. 1) in 154 ml frisch dest. Propionitril wird trockener Chlorwasserstoff eingeleitet. Die Temperatur steigt dabei auf 80°C an. Nach ca. 1 Stunde werden nochmals 50 ml dest. Propionitril zugegeben und unter Rühren weitere 4 Stunden Chlorwasserstoff eingeleitet. Der Ansatz wird über Nacht bei Zimmertemperatur kristallin. Das entstandene Imidchlorid wird mit einer Mischung von 250 ml Wasser und 250 ml Äthanol versetzt und für ca. 30 Min auf 80°C erhitzt, bis im Dünnschichtchromatogramm kein Imidchlorid mehr vorhanden ist. Beim Abkühlen im Eisbad kristallisiert IIIa aus. Man saugt die Kristalle ab, die Mutterlauge wird i. Vak. auf 1/3 eingeengt und nochmals abgekühlt. Die Kristallfraktionen werden vereinigt und aus MeOH umkristallisiert.
- Ausbeute:: 142 g (67 % d. Th.) helle Krist., Fp. 113 - 116°C (IIIa) R_{f}-Wert = 0,51 (Cyclohexan/Essigester = 4 : 1)

### Beispiele 1b - 1e

Die Verbindungen IIIb-IIIe werden in analoger Weise, wie in Beispiel 1a beschrieben, dargestellt (vgl. Tab. 2).

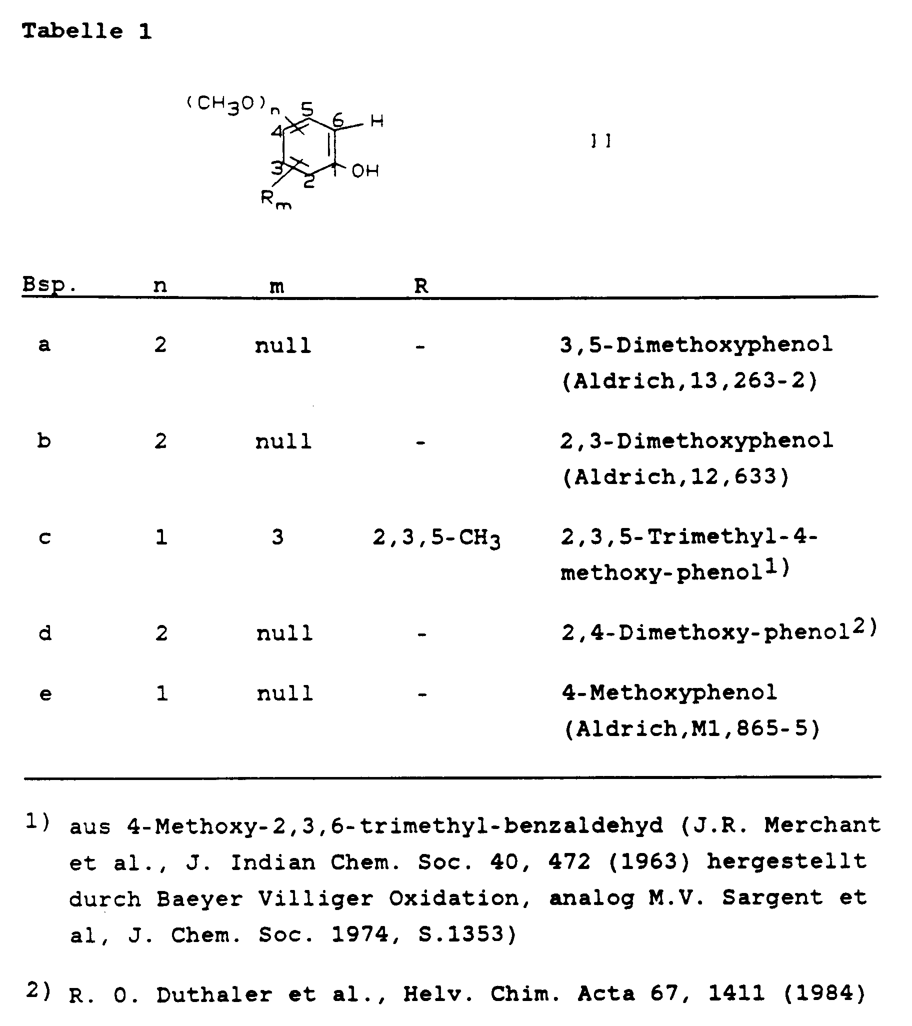

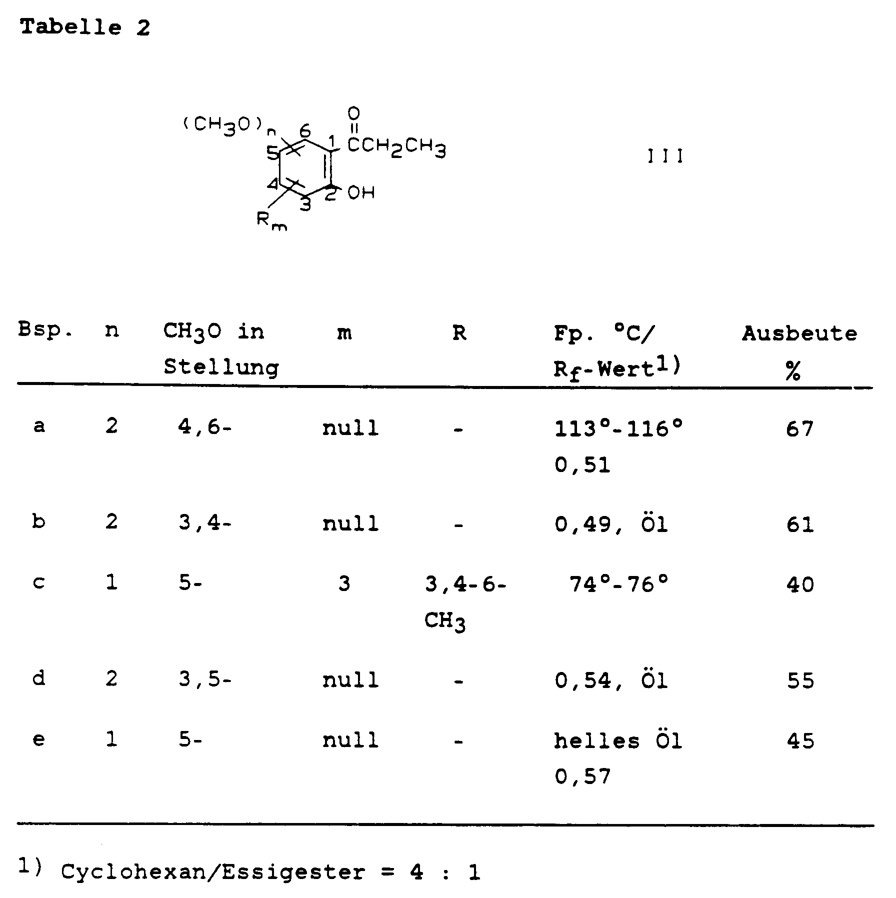

### Beispiel 2a (m = null, n = 2) R², R⁴ = H, R³ = F

### 2-(4-Fluorphenyl)-oxycarbonyl-4,6-dimethoxy-propiophenon Va

In einem Vierhalskolben werden unter Argon 4,5 g (0,15 Mol) Natriumhydrid (80 %ige Suspension in Öl, Fluka AG) in 150 ml abs. Tetrahydrofuran suspendiert. Bei Zimmertemperatur wird eine, Lösung von 31,5 g (0,15 Mol) 2-Hydroxy-4,6-dimethoxy-propiophenon (IIIa) in 350 ml abs. THF langsam zugetropft. Die Temperatur steigt auf 40°C, es entsteht eine blaue Lösung. Es wird 1 Stunde nachgerührt. Dann werden 23,8 g (0,15 Mol; 17,7 ml) p-Fluorbenzoylchlorid (IVa) (R² = H, R³ = F, R⁴ = H) in 100 ml abs. THF zugetropft. Unter leichter wärmetönung entfärbt sich der Ansatz. Es wird 1 Stunde bei Zimmertemperatur weitergerührt. Das Reaktionsgemisch wird im Vakuum eingeengt, der Rückstand mit Essigester/Wasser extrahiert. Die vereinigten organischen Extrakte werden mit MgSO₄ getrocknet, filtriert und im Vakuum eingeengt.
- Ausbeute:: 60 g, Chromatographie an Kieselgel mit Cyclohexan/Essigester = 4 : 1 ergibt 49 g Va, weiße Kristalle (95 % d. Th.)
Fp. 74°C, R_{f}-Wert = 0,34

### Beispiel 2b - 2f

Die Verbindungen Vb-Vf werden in analoger Weise, wie in Beispiel 2a beschrieben, dargestellt (vgl. Tab. 3).

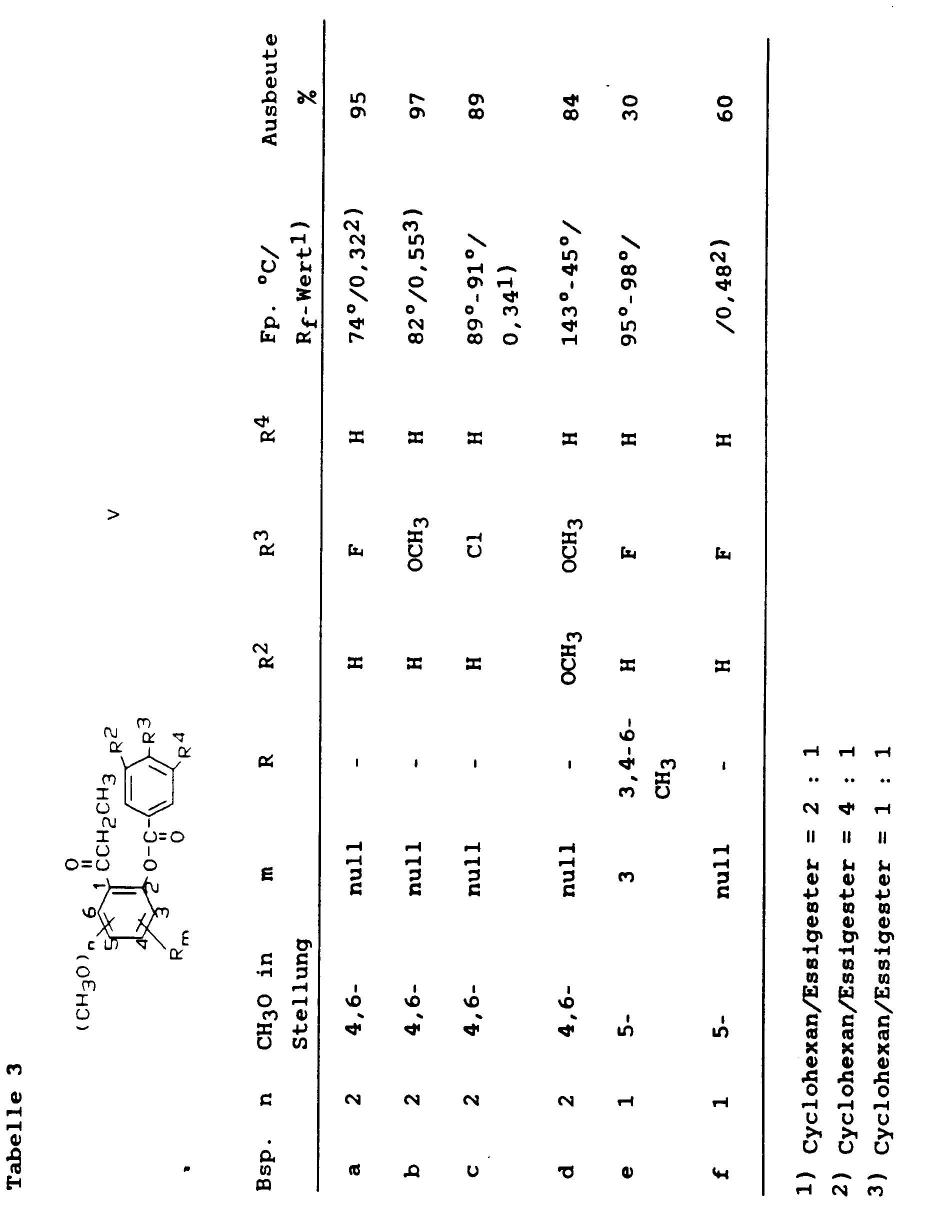

### Beispiel 3a (m = null, n=2, R², R⁴ = H, R⁶ = F)

### 2-(4'-Fluorphenyl)-3-methyl-5,7-dimthoxy-4H-1-benzopyran-4-on (VIa)

Bei Zimmertemperatur werden 0,7 g (17,4 mMol) Natriumhydrid (80 %ige Suspension in Öl) in 20 ml abs. Dimethylsulfoxid in einem Vierhalskolben suspendiert und 30 min bei Zimmertemperatur gerührt. Dann werden unter Argon 1,92 g (5 mMol) 2-(4-Fluorphenyl)-oxycarbonyl-4,6-dimethoxy-propiophenon (Va) in 10 ml abs. DMSO gelöst, zugetropft. Es wird 1 - 2 Stunden bei Zimmertemperatur weitergerührt. Anschließend wird das Reaktionsgemisch bei 0°C mit 50 ml konzentrierter wäßriger Oxalsäurelösung versetzt, 30 Min weitergerührt. Der Ansatz wird mit Essigester extrahiert, die EE-Extrakte werden mit MgSO₄ getrocknet, filtriert und im Vakuum eingeengt. Der Rückstand wird mit 50 ml Eisessig + 2 ml konzentrierter Salzsäure 1 - 2 Stunden am Rückfluß erhitzt, anschließend wird im Vakuum eingeengt. Der Rückstand wird in Methylenchlorid gelöst und mit kalter gesättigter Natriumbicarbonatlösung gewaschen, die CH₂Cl₂-Phase wird mit MgSO₄ getrocknet und im Vakuum eingeengt.
- Ausbeute:: 0,91g weiße Kristalle VIa, (58 % d. Th.)
Fp. 252 - 256°C
R_{f}-Wert = 0,36 (Cyclohexan/Essigester = 1 : 1)

### Beispiel 3b - 3f

Die Verbindungen VIa-VIf werden in analoger Weise, wie in Beispiel 3a beschrieben, dargestellt (vgl. Tab. 4).

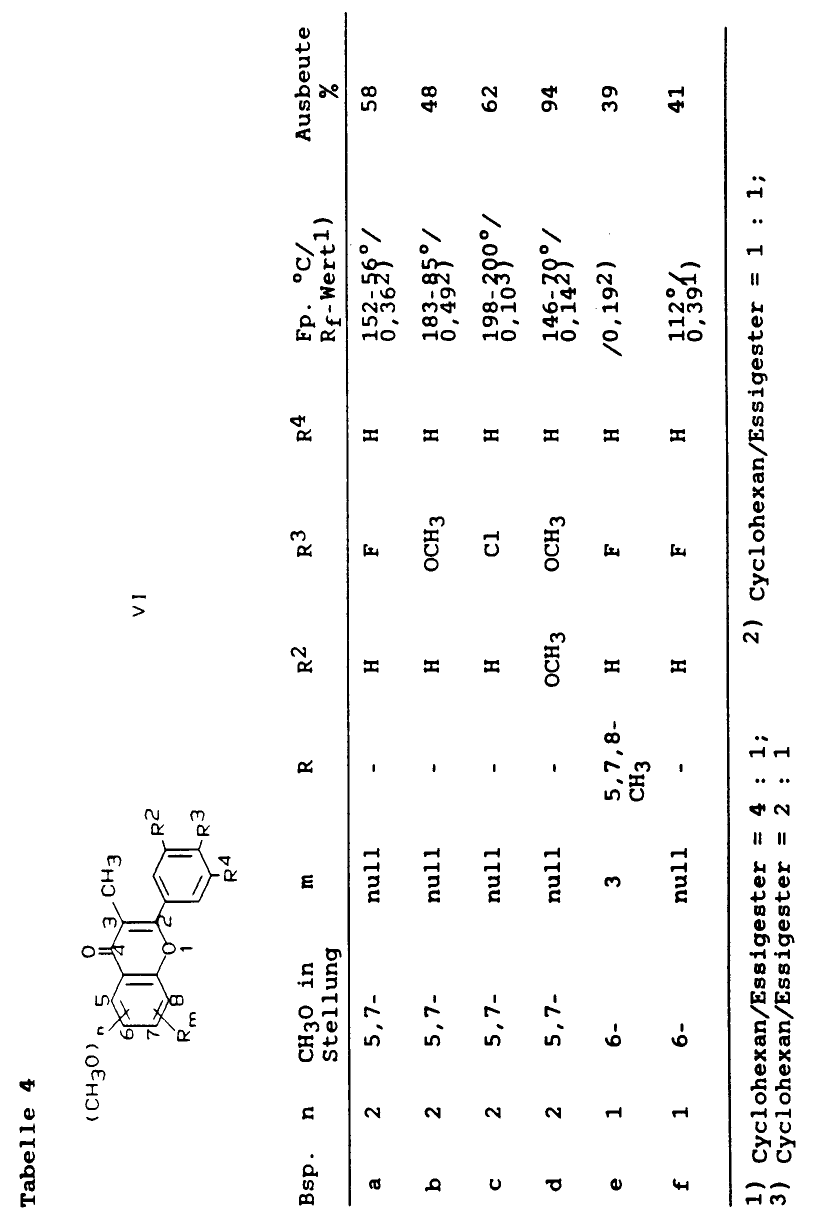

### Beispiel 4a (m = null, n=2, R², R⁴ = H, R³ = F)

### 2-(4'-Fluorphenyl-3-methyl-5,7-dihydroxy-4H-1-benzopyran-4-on (VIIa)

0,9 g (2,9 mMol) 2-(4'-Fluorphenyl-3-methyl-5,7-dimethoxy-4H-1-benzopyran-4-on (VIa) werden in 3,5 ml Jodwasserstoffsäure (57 %ig) 1 Stunde unter Argon auf 110°C erwärmt. Anschließend wird das Reaktionsgemisch zu einer Mischung von 50 ml H₂O und 50 ml Essigester gegeben. Die Essigesterphase wird abgetrennt und 1 x mit Wasser und 1 x mit gesättigter Natriumbicarbonatlösung säurefrei gewaschen, mit MgSO₄ getrocknet, filtriert und im Vakuum eingeengt. Am Kieselgel wird mit Cyclohexan/Essigester = 4 : 1 als Elutionsmittel chromatographiert.
- Ausbeute:: 0,63 g schwach gelbe Kristalle VIIa (82 % d. Th.)
Fp. = 276 - 279°C
R_{f}-Wert = 0,74 (Cyclohexan/Essigester = 1 : 1)

### Beispiel 4b - 4g

Die Verbindungen VIIb-VIIg werden in analoger Weise, wie in Beispiel 4a beschrieben, dargestellt (vgl. Tab. 5).

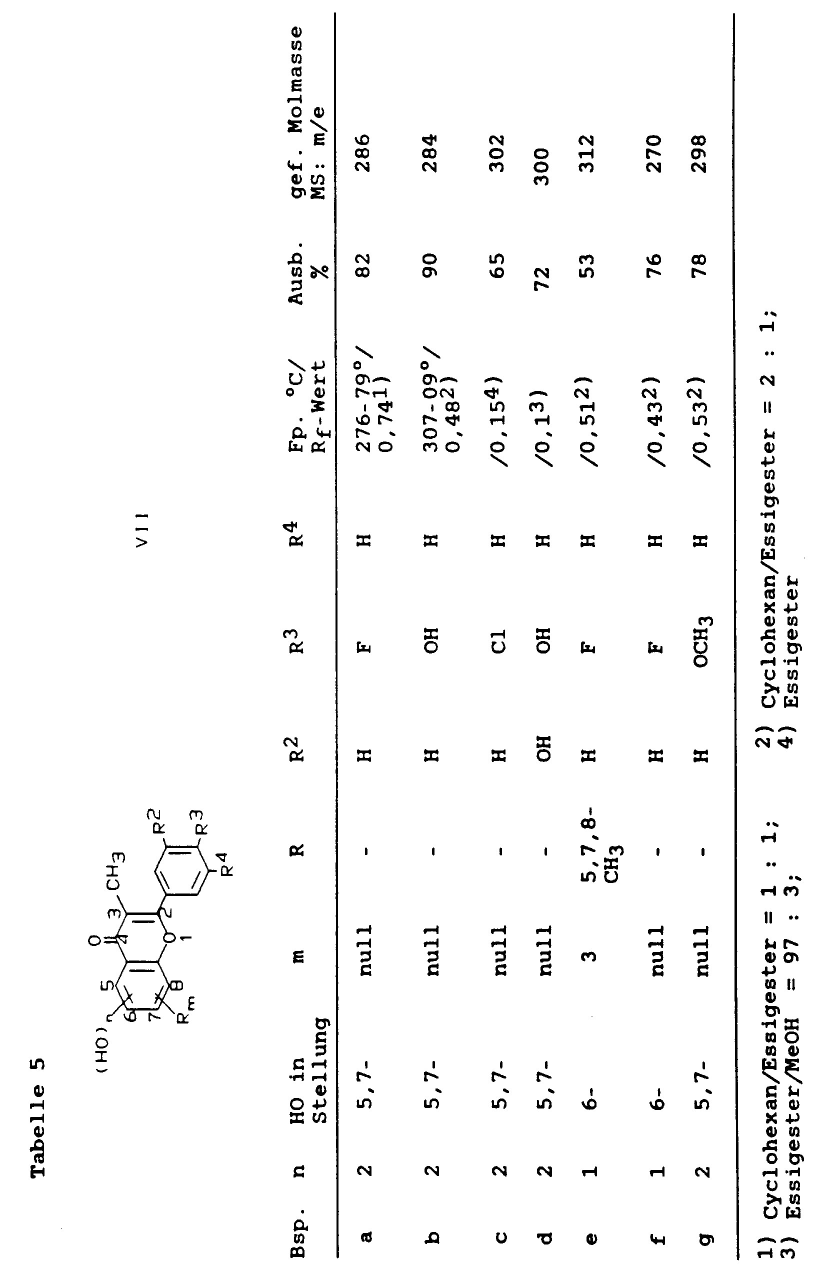

### Beispiel 5a (m = null, n=2, R^{2'}, R^{4'} = H, R^{3'} = F)

### 2-(4'-Fluorphenyl)-3-methyl-5,7-bisacetoxy-4H-1-benzopyran-4-on (VIIIa)

Unter Feuchtigkeitsausschluß werden 0,63 g (2,2 mMol) 2-(4'-Fluorphenyl)-3-methyl-5,7-dihydroxy-4-H-1-benzopyran-4-on VIIa in 8 ml Essigsäureanhydrid gelöst. Nach Zugabe von 1,5 ml Pyridin abs. wird 2 Stunden auf 100°C erwärmt. Dann wird das Reaktionsgemisch mit einer 1 : 1 Mischung von 50 ml Essigester/Eiswasser versetzt und extrahiert. Die Wasserphase wird noch 2 x mit Essigester extrahiert. Die vereinigten organischen Extrakte werden mit MgSO₄ getrocknet, filtriert und im Vakuum eingeengt. An Kieselgel 60 µm Merck AG, Darmstadt wird mit Cyclohexan/Essigester = 4 : 1 als Elutionsmittel chromatographiert.
- Ausbeute:: 0,81 g helle Kristalle Villa (98 % d. Th.)
Fp. = 147 - 149°C
R_{f}-Wert = 0,35 (Cyclohexan/Essigester = 1 : 1)

### Beispiel 5b - 5g

Die Verbindungen VIIIa-VIIIg werden in analoger Weise, wie in Beispiel 5a beschrieben, dargestellt (vgl. Tab. 6).

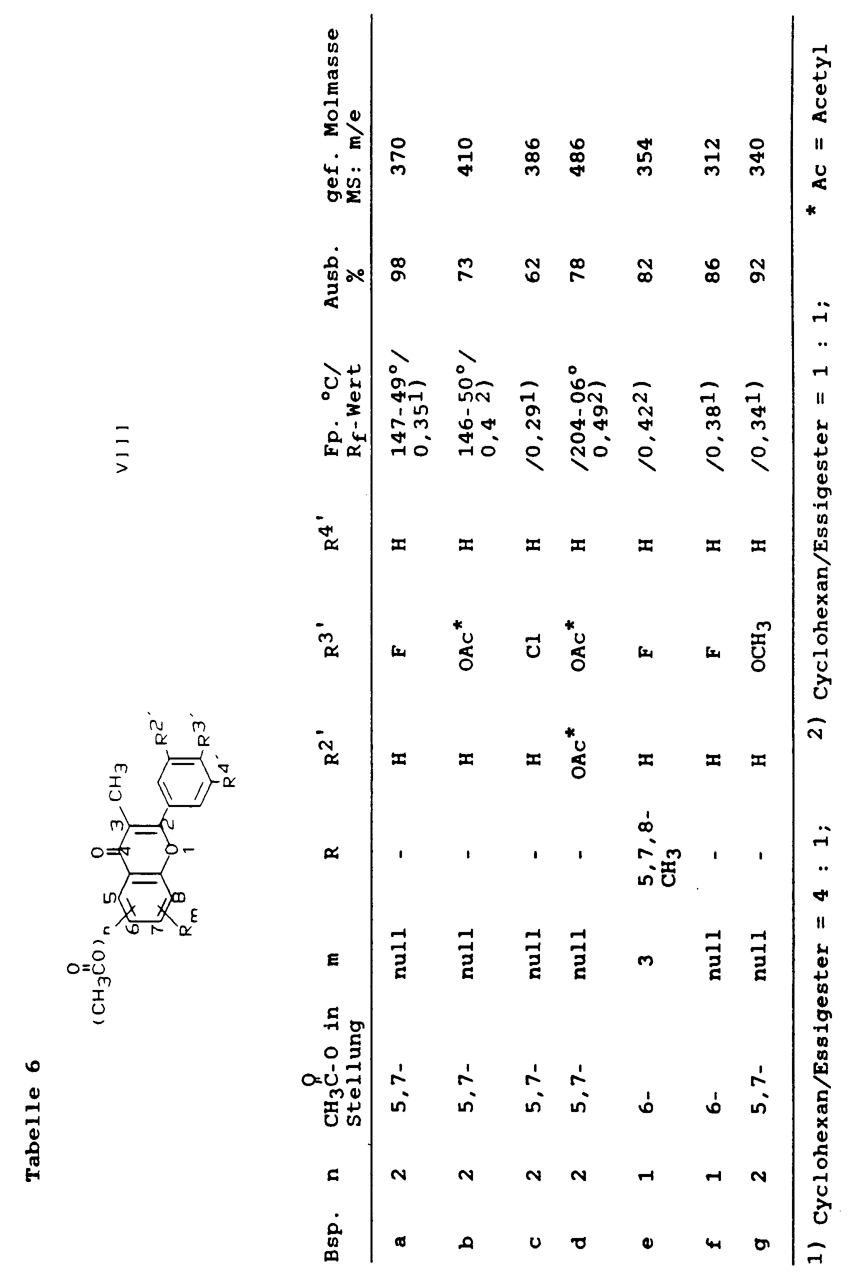

### Beispiel 6a (m = null, n=2, R^{2'}, R^{4'} = H, R^{3'} = F, Hal = Brom, Ac = Acetyl)

### 2-(4'-Fluorphenyl)-3-brommethyl-5,7-bisacetoxy-4H-1-benzopyran-4-on (IXa)

2,8 g (7,4 mMol) 2-(4'-Fluorphenyl)-3-methyl-5,7-bis-acetoxy-4H-1-benzopyran-4-on (VIIIa) werden in 50 ml Tetrachlor-Kohlenstoff abs. gelöst. Nach Zugabe von 0,88 g (4,8 mMol) N-Bromsuccinimid und 0,1 g N,N-Azobis-(isobutyronitril) wird 3 - 4 Stunden unter Feuchtigkeitsausschluß am Rückfluß erhitzt. Nach Zugabe weiterer 0,44 g (2,4 mMol) N-Bromsuccinimid wird 4 Stunden am Rückfluß gekocht. Nach Abkühlung wird vom ausgefallenen Succinimid abfiltriert. Das Filtrat wird eingeengt und der Rückstand aus Essigester umkristallisiert.
- Ausbeute:: 2,5 g weiße Kristalle IXa (76 % d. Th.)
Fp. 167 - 170°C
R_{f}-Wert: 0,64 (Toluol/Essigester = 4 : 1)

### Beispiel 6b - 6g

Die Verbindungen IXb - IXg werden in analoger Weise, wie in Beispiel 6a beschrieben, dargestellt (vgl. Tab. 7).

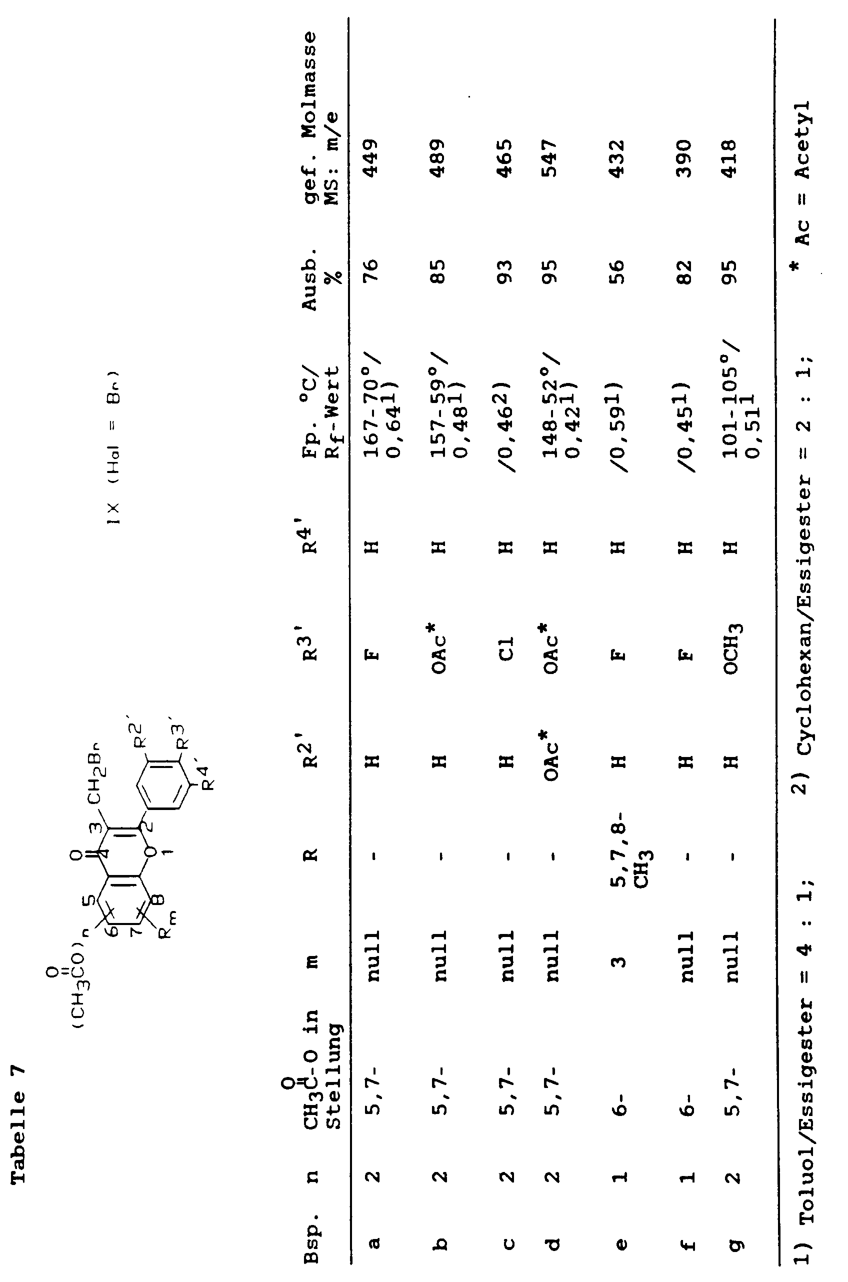

### Beispiel 7a (R¹ = Phytyl, X = S)

### 3,7R,11R,15-Tetramethyl-2E-hexadecenyl-mercaptan (Xa)

A) 9,5 g (33 mMol) 3,7R,11R,15-Tetramethyl-2E-hexadecen-1-ol (Phytol, Aldrich 13, 991-2) werden in 30 ml abs. Methylenchlorid gelöst. Bei 0°C werden 2,1 ml Phosphortribromid zugetropft. Dann wird 3 Stunden bei 0°C weitergerührt. Der Reaktionsansatz wird auf 50 ml Eiswasser gegeben und mit Methylenchlorid extrahiert. Die Methylenchloridextrakte werden mit verd. NaHCO₃-Lösung säurefrei gewaschen, mit MgSO₄ getrocknet, filtriert und i. Vak. eingeengt. Das erhaltene Phytylbromid (R¹-Br)(11,0 g; 92,8 % d. Th.) wird direkt weiter umgesetzt.
B) 2,5 g (33 mMol) Thioharnstoff werden in 25 ml Äthanol abs. gelöst. Dazu gibt man 11,0 g (30,5 mMol) Phytylbromid (R¹-Br) aus Ansatz A) in 25 ml abs. Äthanol gelöst. Es wird 6 Stunden unter Rückfluß erhitzt. Beim Abkühlen fällt das gebildete Isothiuroniumsalz aus. Das Salz kann abgesaugt werden oder es wird direkt weiterverarbeitet. Dazu gibt man 10 ml 5N Natronlauge zum Ansatz und erhitzt 2 Stunden am Rückfluß. Nach dem Abkühlen wird mit 2N Salzsäure auf pH 4 gestellt und das Mercaptan mit Diäthyläther (jeweils 50 ml) 3 x extrahiert. Die Ätherextrakte werden mit MgSO₄ getrocknet, filtriert und i. Vak. eingeengt. Chromatographie an Kieselgel Merck 60 µm, mit Cyclohexan/Essigester 10 : 1 ergibt Xa.

- Ausbeute:: 5,9 g helles Öl Xa (58 % d. Th.)
R_{f}-Wert = 0,73 (Cyclohexan/Essigester = 4 : 1)

### Beispiel 7b - 7f,

Die Verbindungen Xb - Xf, werden in analoger Weise aus den käuflichen Alkoholen, wie in Beispiel 7a beschrieben, hergestellt. (vgl. Tab. 8).

### Beispiel 7g

Xg wird durch Addition von Äthanol an Xe erhalten.

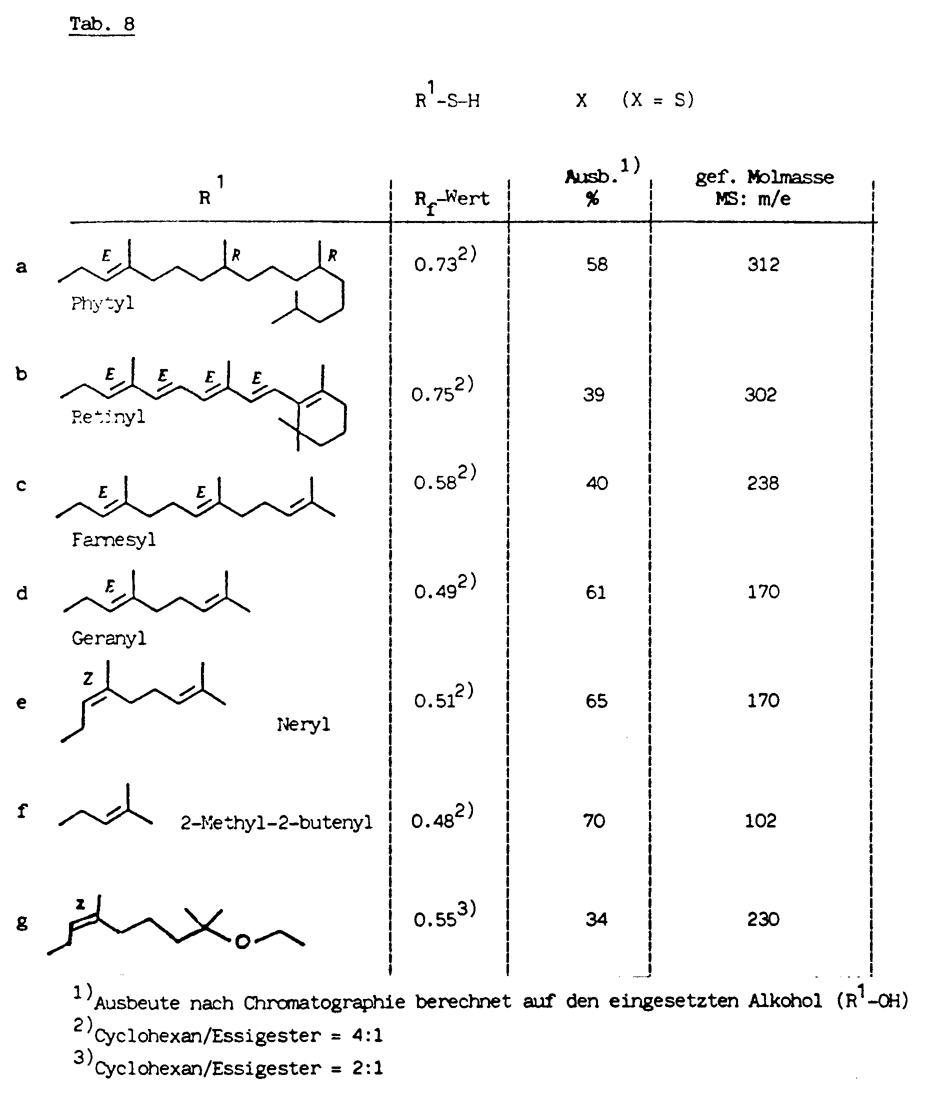

### Beispiel 8a (m = null, n = 2, R^{2'}, R^{4'} = H, R^{3'} = F, X = S) (Vergleichsbeispiel)

### 2-(4'-Fluorphenyl)-3-(2-thia-octadecyl)-5,7-bis-acetoxy-4H-1-benzopyran-4-on (XIa)

0,9 g (2 mMol) 2-(4'-Fluorphenyl)-3-brommethyl-5,7-bisacetoxy-4H-1-benzopyran-4-on (IXa) in 20 ml abs. Tetrahydrofuran werden zu einer Suspension von Natrium-Cetylmercaptan (hergestellt aus 0,62 g (2,5 mMol) Cetylmercaptan (Aldrich H 763-7) und O,11 g (2,5 mMol) Natriumhydrid (55 %ige Suspension in Öl) in 20 ml abs. Tetrahydrofuran) bei Zimmertemperatur zugegeben. Man rührt ca. 3 Stunden unter Argon. Anschließend wird der Reaktionsansatz mit 50 ml Essigester und 50 ml Wasser versetzt und extrahiert. Die org. Extrakte werden mit MgSO₄ getrocknet, filtriert und i. Vak. eingeengt. Der Rückstand wird an Kieselgel Merck 60 µm mit Cyclohexan/Essigester = 8 : 2 chromatographiert.
- Ausbeute:: 1,05 g gelbe Kristalle XIa (83 % d. Th.)
MS: gef. Molmasse 626
R_{f}-Wert: 0,95 (Cyclohexan/Essigester = 2 : 1)

### Beispiel 8b - 8y

Die Verbindungen XIb-XIy werden in analoger Weise, wie unter Beispiel 8a beschrieben, hergestellt. Für alle Beispiele bei denen X = Sauerstoff bedeutet, werden anstelle der Mercaptane R¹-SH, die entsprechenden käuflichen Alkohole R¹-OH eingesetzt, ansonsten aber wie unter Beispiel 8a beschrieben, umgesetzt (vgl. Tab. 9).

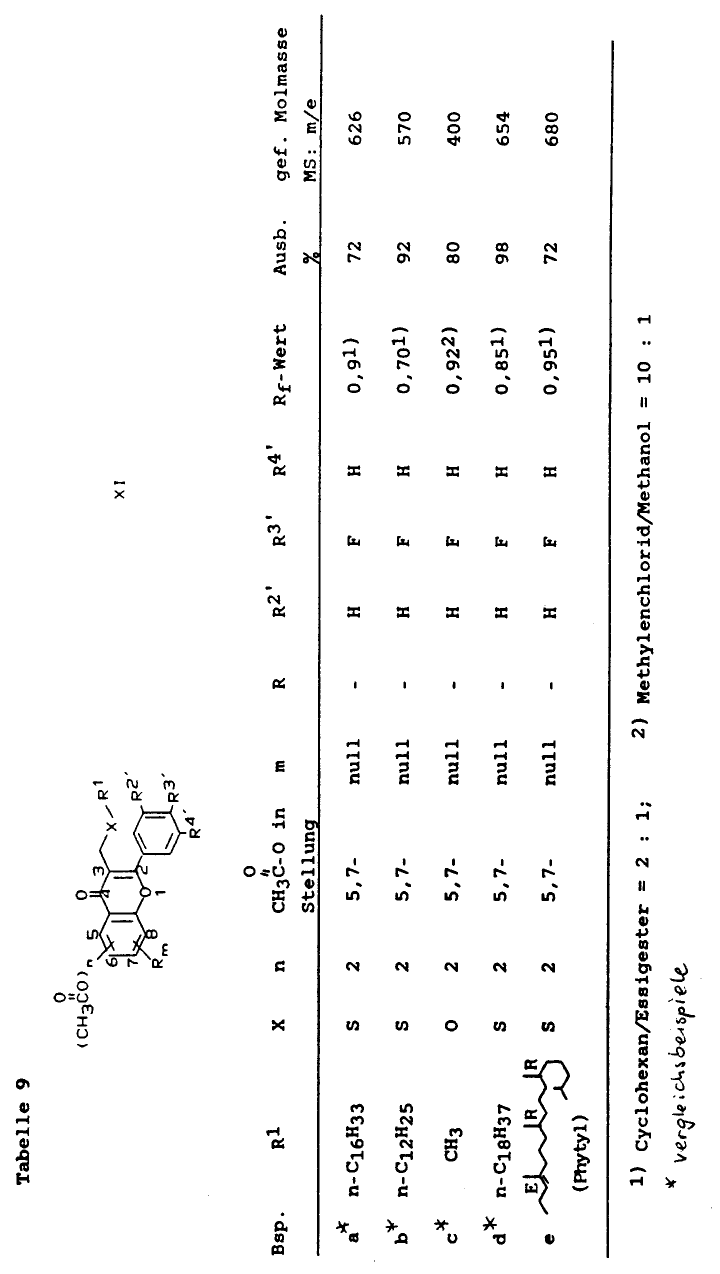

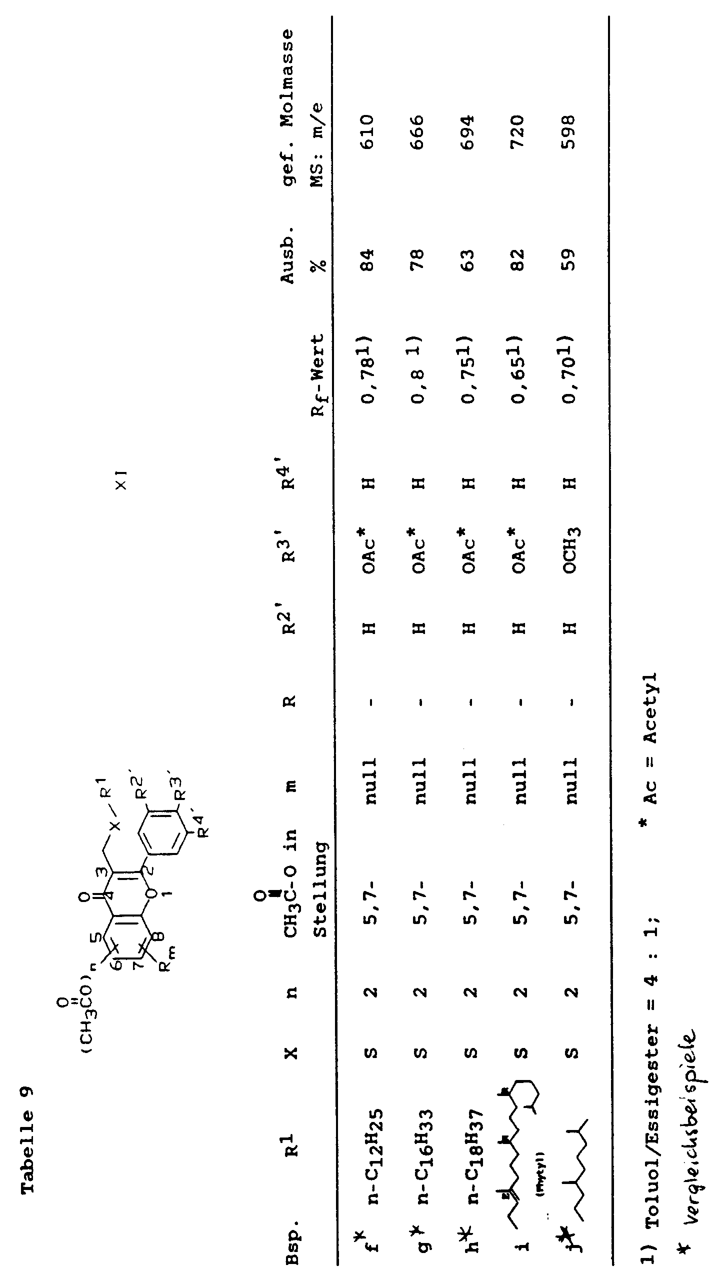

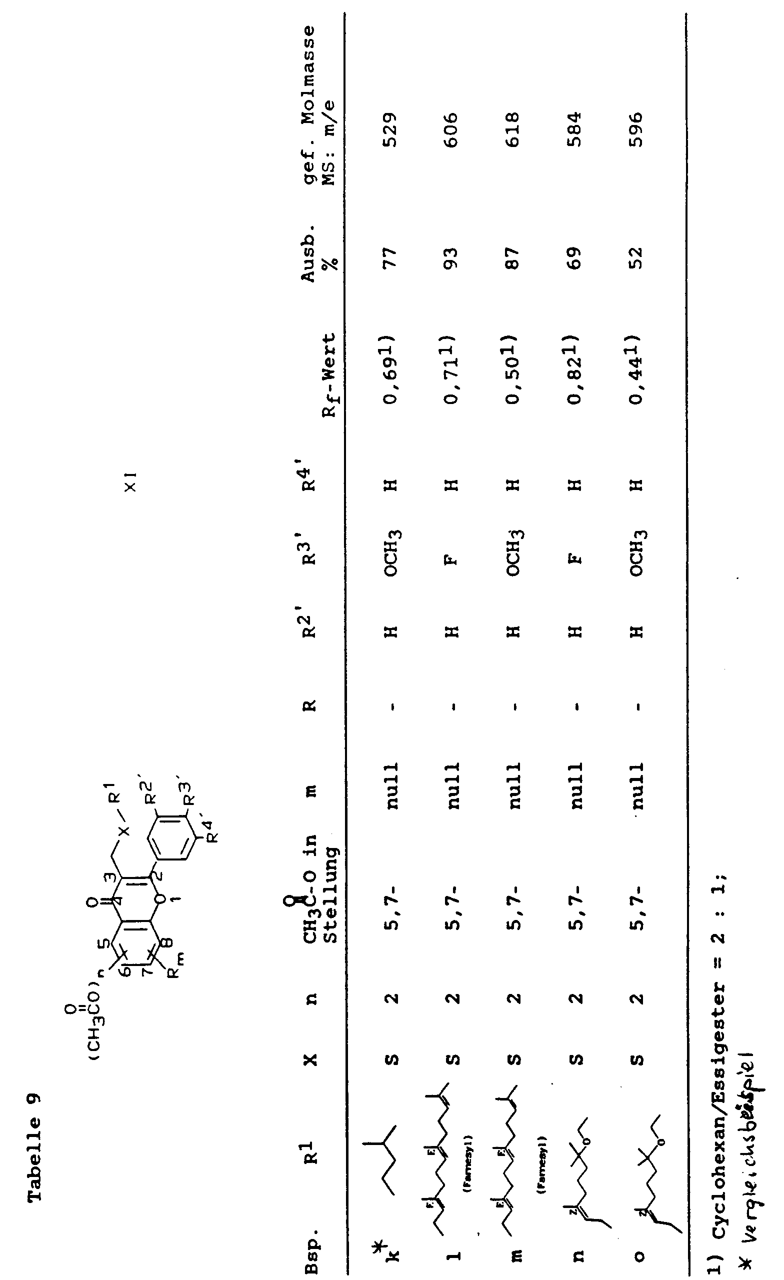

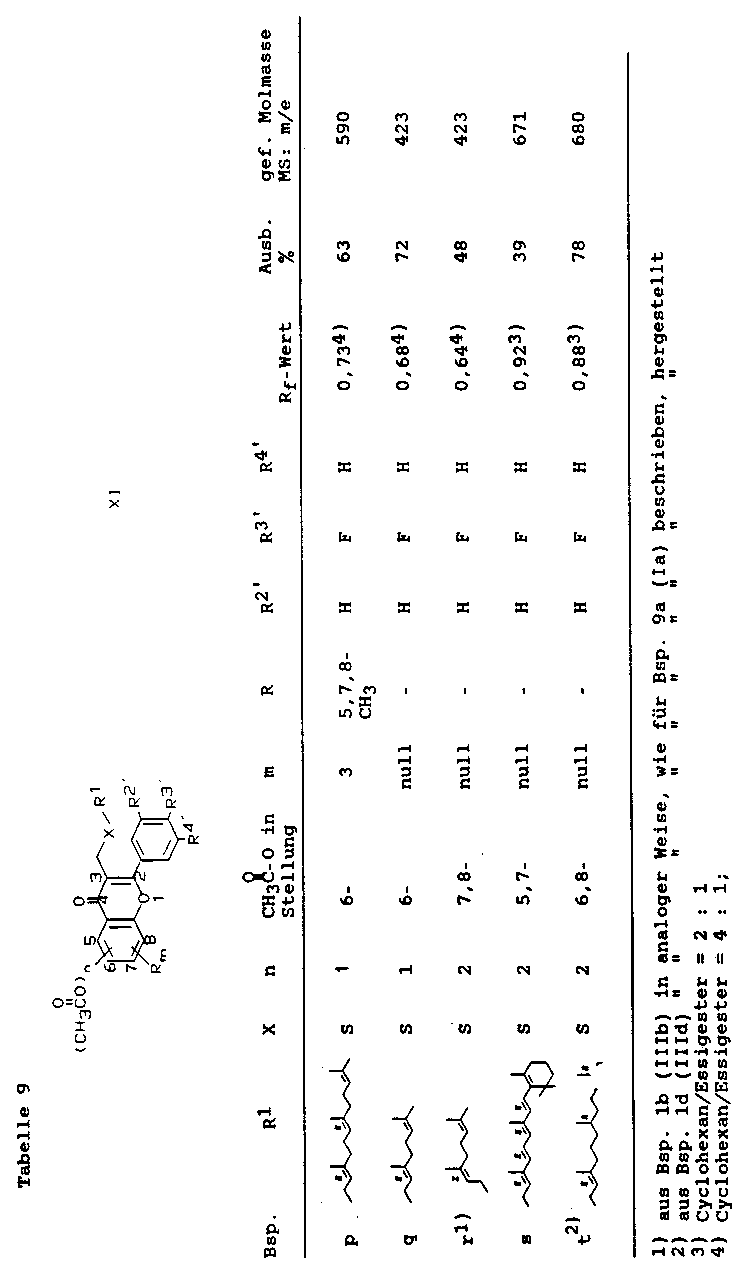

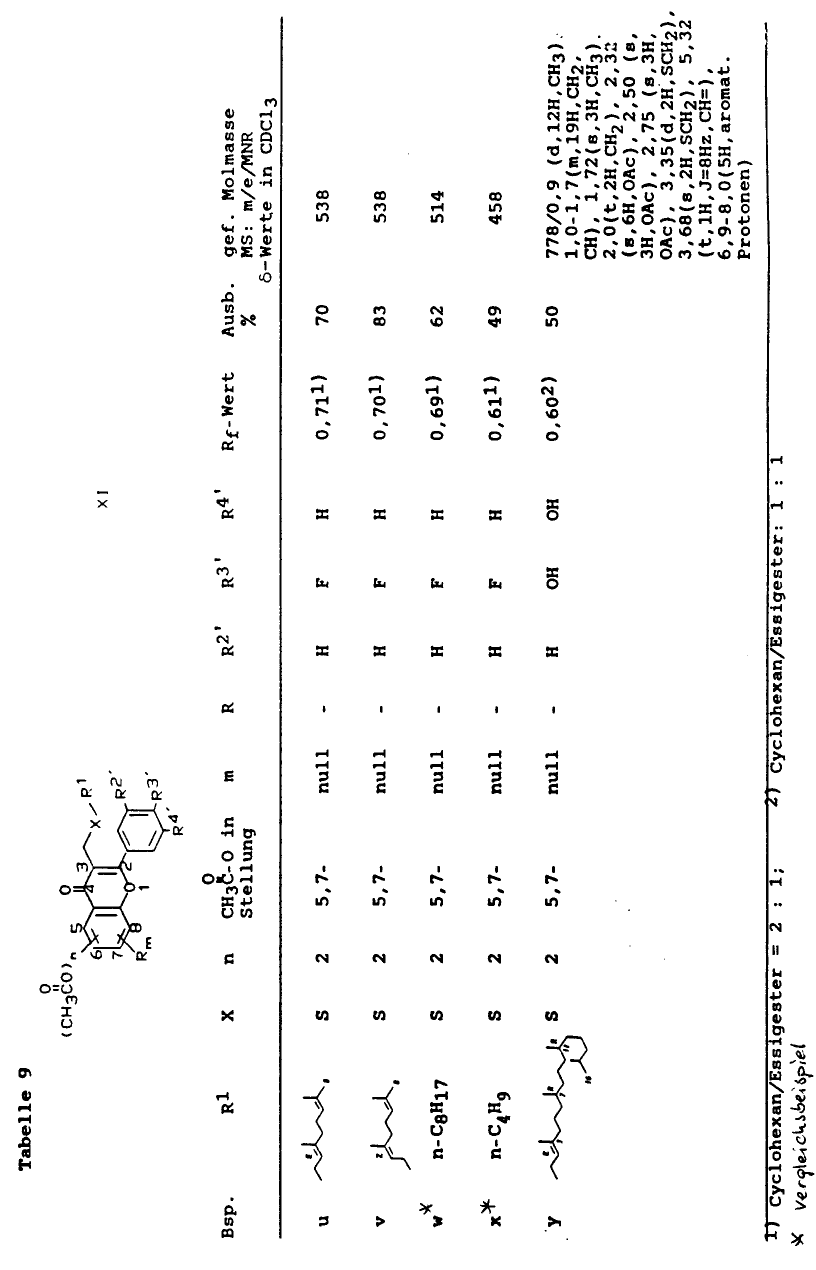

### Beispiel 9a (m = null, n = 2, R², R⁴ = H, R³ = F, X = S)

### 2-(4'-Fluorphenyl)-3-(2-thia-octadecyl)-5,7-dihydroxy-4-H-1-benzopyran-4-on Ia (Vergleichsbeispiel)

0,8 g (1,3 mMol) 2-(4'-Fluorphenyl)-3-(2-thia-octadecyl)-5,7-bisacetoxy-4H-1-benzopyran-4-on (XIa) werden in 10 ml Methanol gelöst. Nach Zugabe von 0,37 g (27 mMol) pulverisiertem Kaliumcarbonat wird 2 Stunden bei Zimmertemperatur gerührt. Der Reaktionsansatz wird mit 30 ml Essigester und 40 ml Wasser versetzt und extrahiert. Die org. Extrakte werden mit MgSO₄ getrocknet, abfiltriert und im Vakuum eingeengt.
- Ausbeute:: 0,6 g helle Kristalle (86 % d. Th.) Ia MS: gef. Molmasse 542
R_{f}-Wert: 0,61 (Cyclohexan/Essigester = 2 : 1)
Fp: 110 - 112°C

### Beispiele 9b - 9y

Die Verbindungen Ib - Iy werden in analoger Weise, wie unter Beispiel 9a beschrieben, hergestellt (vgl. Tab. 10).

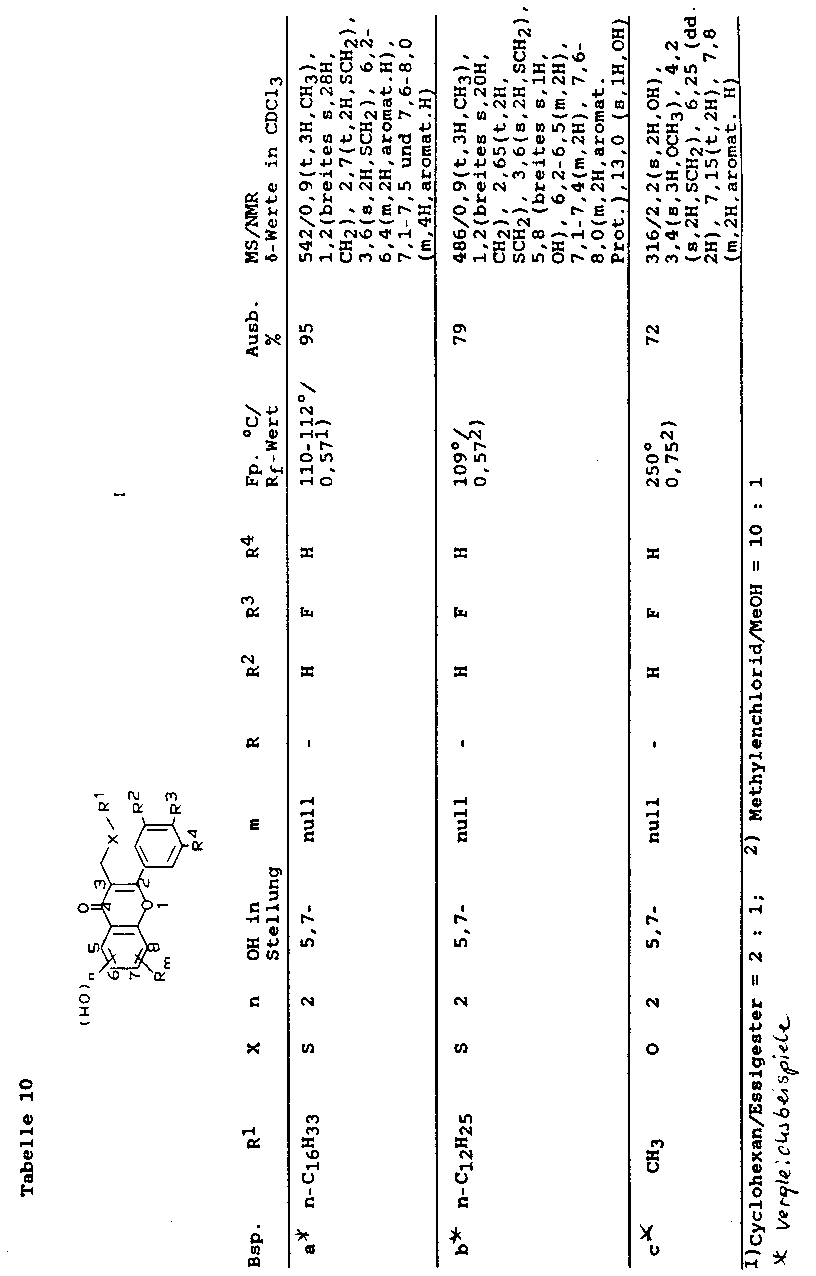

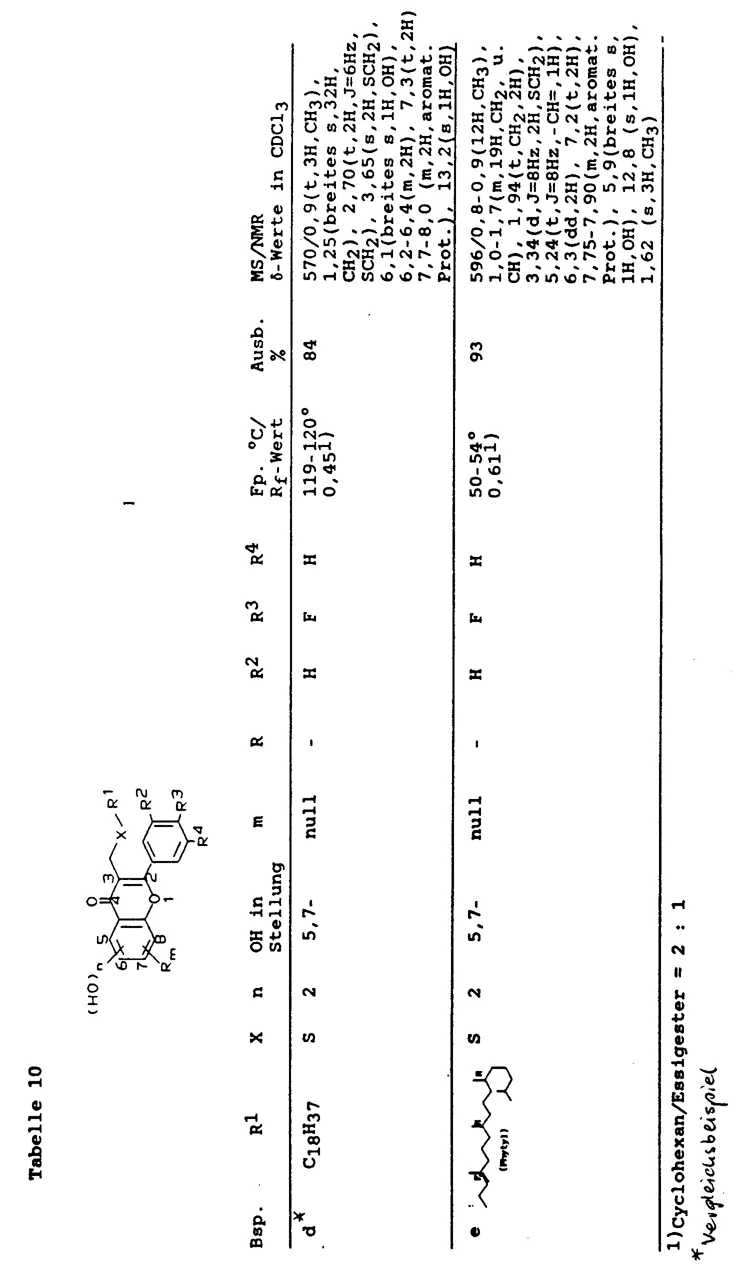

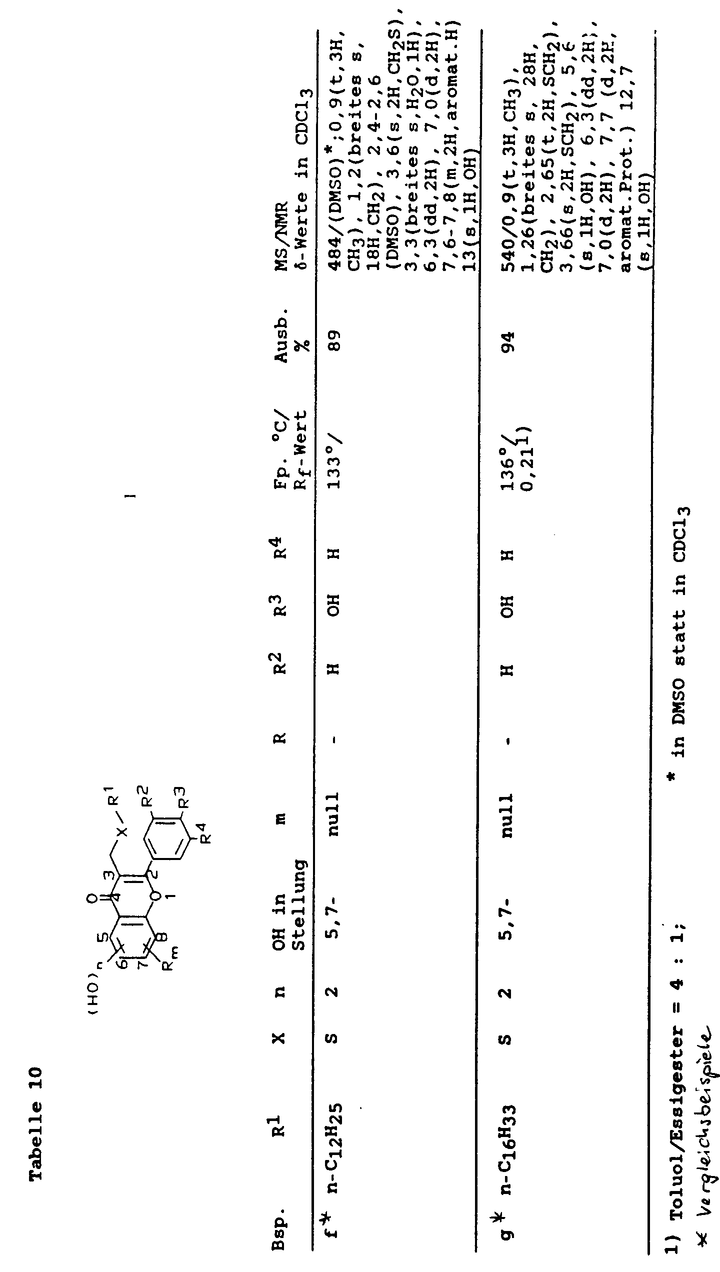

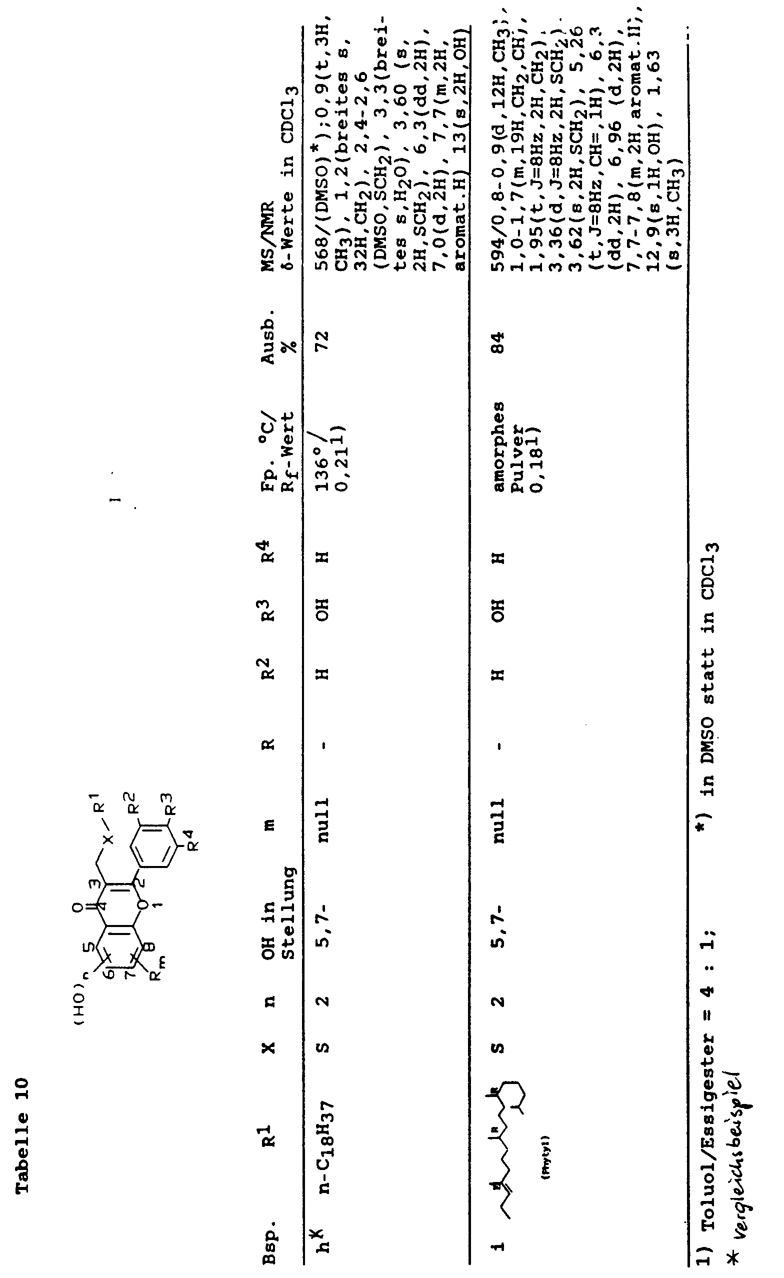

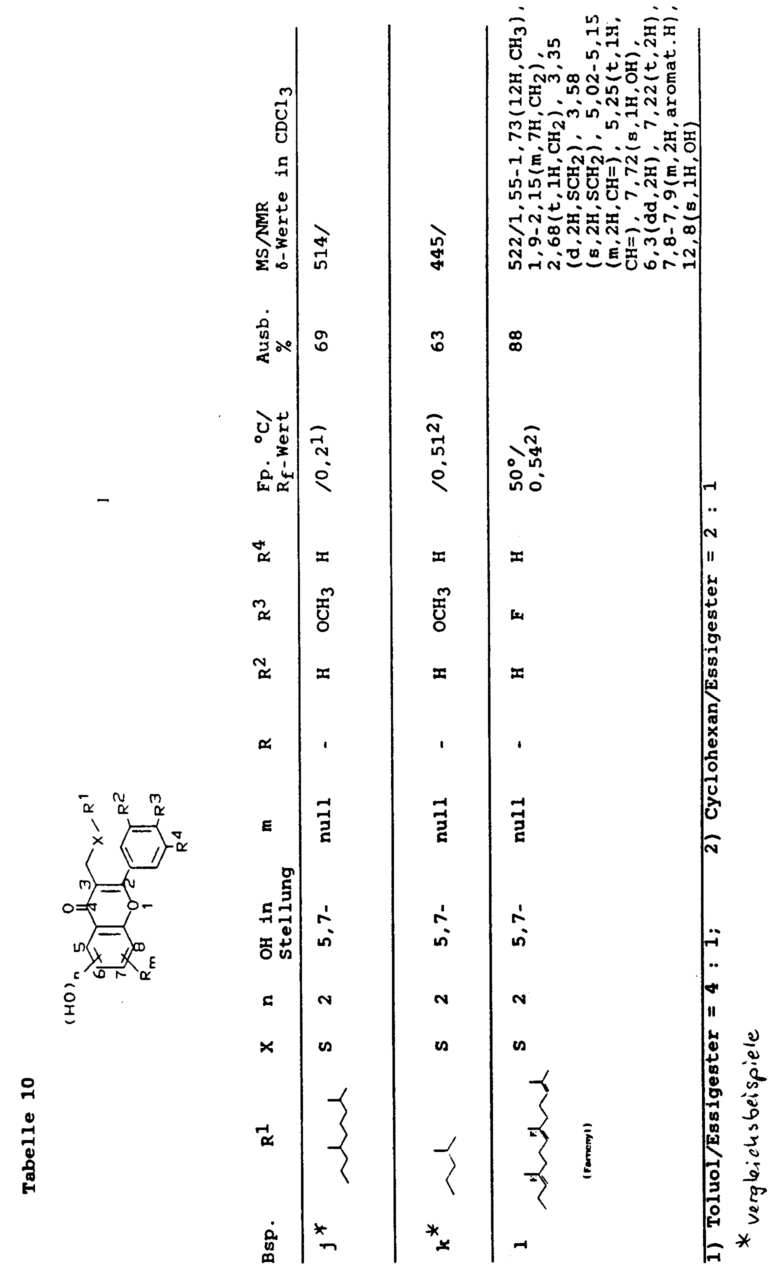

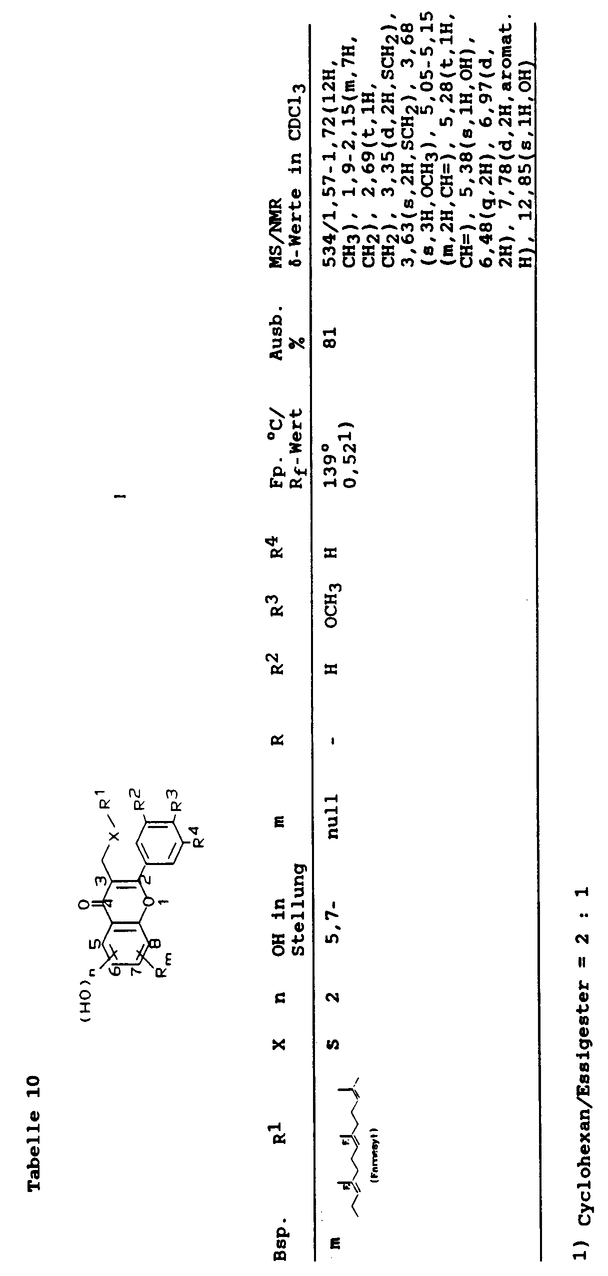

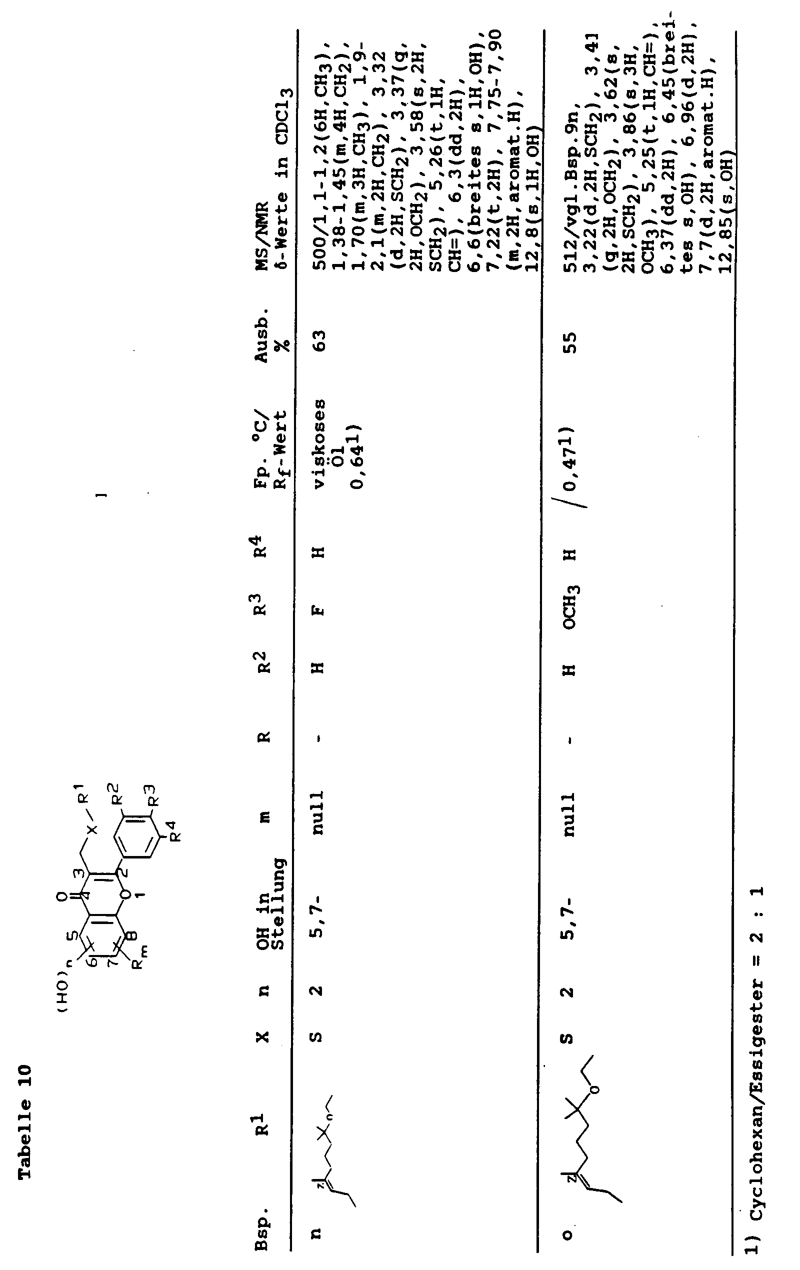

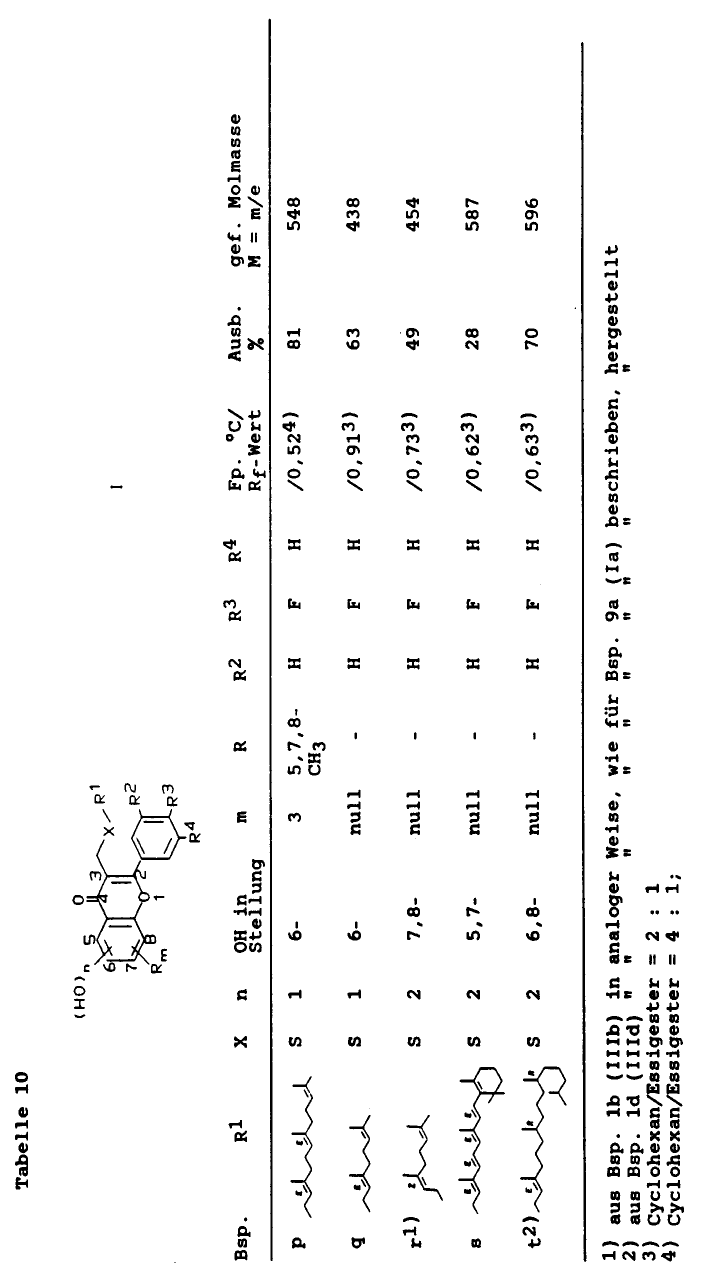

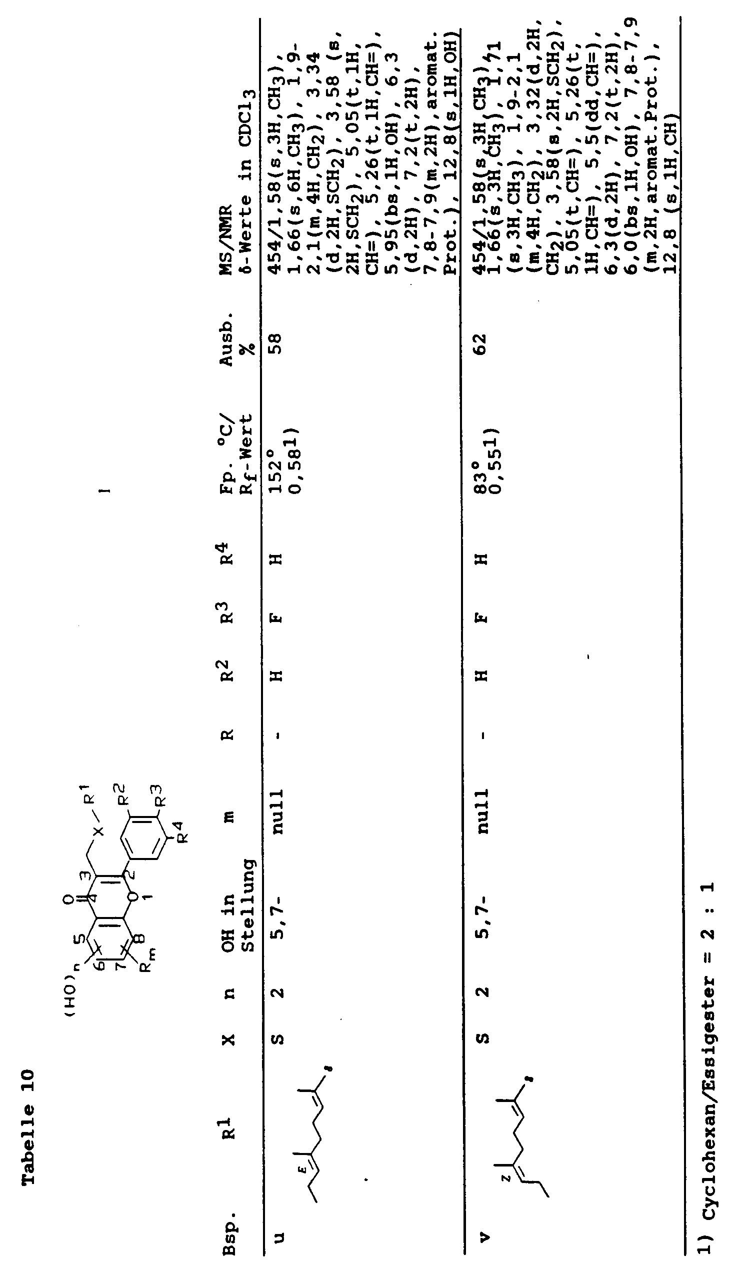

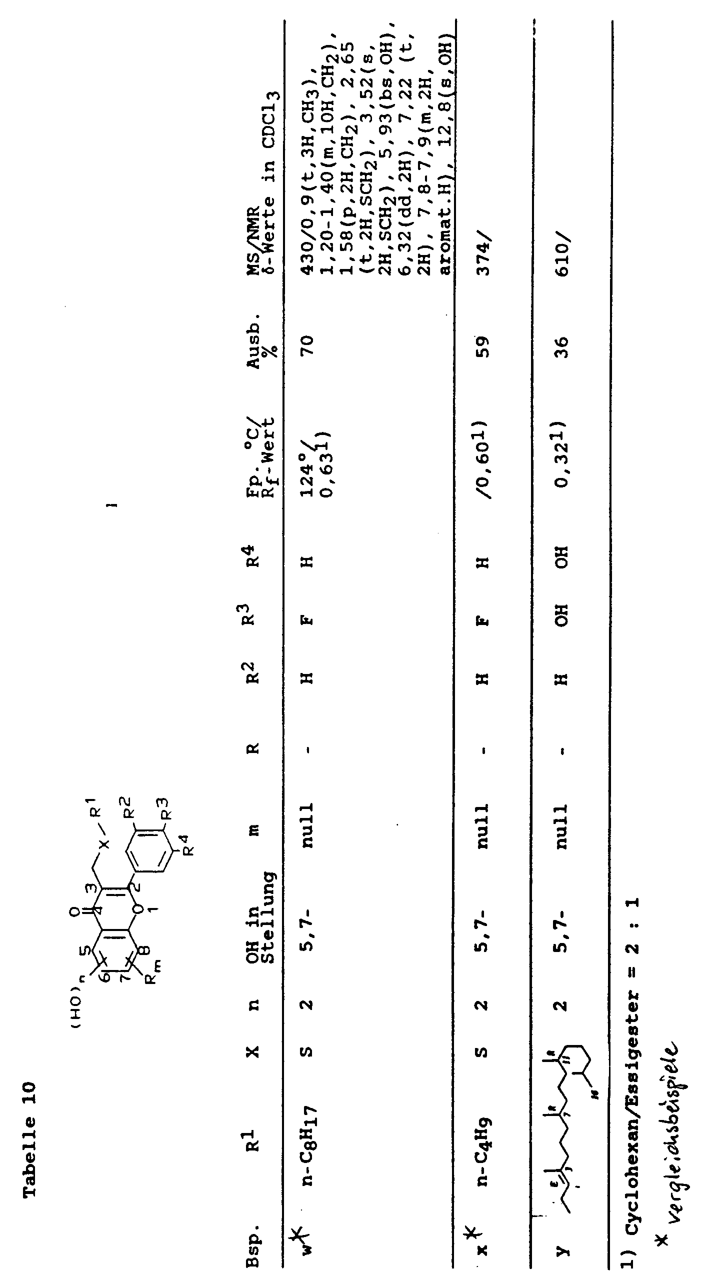

## Patentansprüche (Patentansprüche für folgende(n) Vertragsstaat(en): AT, BE, CH, DE, DK, FR, GB, IT, LI, LU, NL, SE)

1. Substituierte 3-Thia- bzw. 3-Oxa-alkyl-Flavone der Formel I, 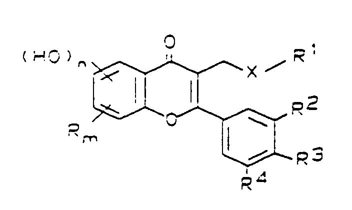 in der bedeuten:
X Schwefel oder Sauerstoff
R Halogen, (C₁-C₄)-Alkyl oder Trifluor-methyl,
m null, 1, 2 oder 3,
n 1 oder, falls m = null, 1 oder 2 ist, auch 2,
R¹ (C₃-C₂₅)-Alkenyl, wobei gegebenenfalls eine CH₂-Gruppe durch Sauerstoff ersetzt ist, oder (C₃-C₂₅)-Alkenyl, das mit Cyclohexenyl, das seinerseits 1 - 3 Methylgruppen enthält, substituiert ist,
R²,R³,R⁴ Wasserstoff, Hydroxy, Halogen, (C₁-C₄)-Alkyl, (C₁-C₄)-Alkoxy, Trifluormethyl, wobei R², R³, R⁴ gleich oder verschieden sind.

2. Verfahren zur Herstellung der Verbindungen der Formel I, dadurch gekennzeichnet, daß man
a) ein Phenol der Formel II 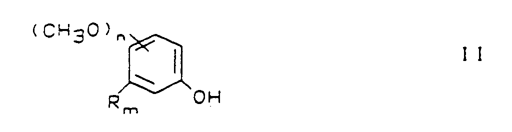 worin m, n und R die zur Formel I angegebene Bedeutung haben,
durch Acylierung in die Verbindungen der Formel III überführt 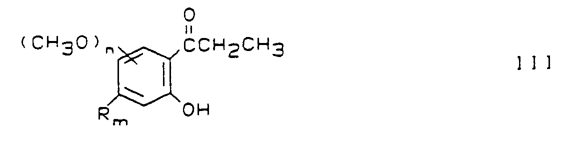 worin m, n und R die zur Formel I angegebene Bedeutung haben,
b) eine Verbindung der Formel III an der freien phenolischen Hydroxylgruppe mit einem Säurehalogenid der Formel IV, 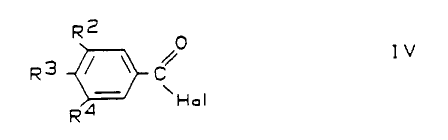 worin R², R³, R⁴ die zur Formel I angegebene Bedeutung haben und Hal gleich Brom oder Chlor ist, acyliert zu einer Verbindung der Formel V 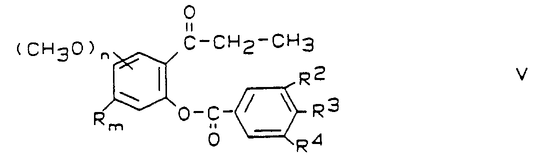 worin m, n, R und R², R³, R⁴ die zur Formel I angegebene Bedeutung haben,
c) eine Verbindung der Formel V cyclisiert Zu einer Verbindung der Formel VI 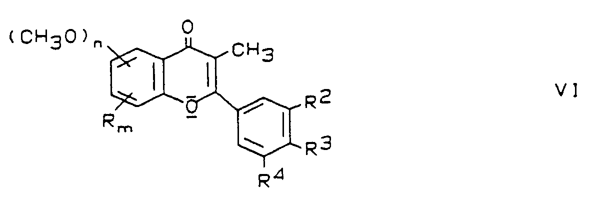 worin m, n, R und R², R³, R⁴ die zur Formel I angegebene Bedeutung haben,
d) in einer Verbindung der Formel VI die Methoxygruppen abspaltet, zu einer Verbindung der Formel VII 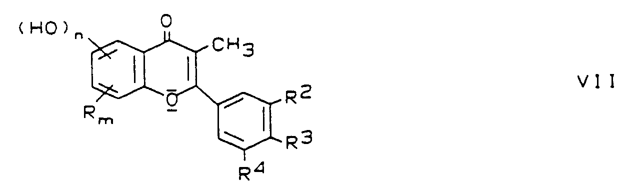 worin m, n und R sowie R², R³, R⁴ die zur Formel I angegebene Bedeutung haben,
e) eine Verbindung der Formel VII mit einem Acylierungsmittel zu einer Verbindung der Formel VIII umsetzt, 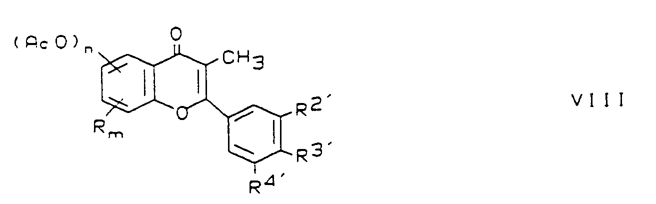 worin m, n und R die zur Formel I angegebene Bedeutung haben, und R^{2'}, R^{3'}, R^{4'} Wasserstoff, Halogen, (C₁-C₄)-Alkyl, (C₁-C₄)-Alkoxy, Trifluormethyl oder Acyloxy sind, wobei R^{2'}, R^{3'}, R^{4'} gleich oder verschieden sind,
f) eine Verbindung der Formel VIII selektiv halogeniert zu einer Verbindung der Formel IX 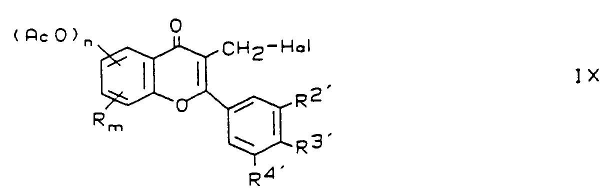 worin m, n und R die zur Formel I, R^{2'}, R^{3'}, R^{4'} die zur Formel VIII angegebene Bedeutung haben und Hal gleich Chlor, Brom oder Jod ist,
g) eine Verbindung der Formel IX umsetzt mit einer Verbindung der Formel X
H-X-R¹ X
worin X und R¹ die zur Formel I angegebene Bedeutung haben,
zu einer Verbindung der Formel XI 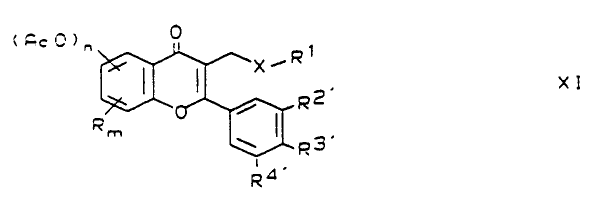 worin m, n, X, R und R¹ die zur Formel I und R^{2'}, R^{3'}, R^{4'} die zur Formel VIII angegebene Bedeutung haben,
h) in einer Verbindung der allgemeinen Formel XI die Acylschutzgruppen abspaltet zu einer Verbindung der allgemeinen Formel I.

3. Pharmazeutisches Präparat, dadurch gekennzeichnet, daß es eine Verbindung gemäß Anspruch 1 enthält.

4. Verbindungen der Formel IX 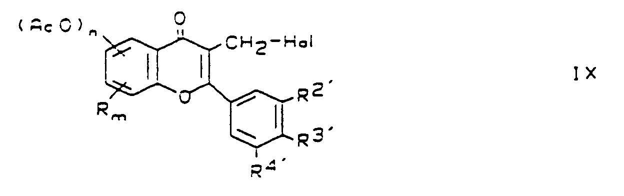 worin m, n und R die zur Formel I in Anspruch 1 und R^{2'}, R^{3'}, R^{4'} die zu Formel VIII in Anspruch 2 (e) angegebene Bedeutung haben.

5. Verbindungen der Formel XI 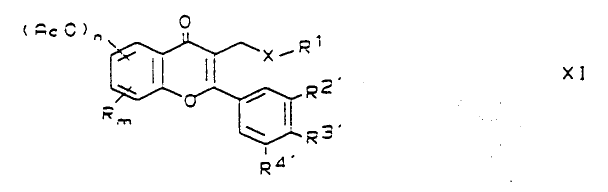 worin m, n, X, R und R¹ die zur Formel I in Anspruch 1 und R^{2'}, R^{3'}, R^{4'} die zur Formel VIII in Anspruch 2 (e) angegebene Bedeutung haben.

6. Verbindungen gemäß Anspruch 1, dadurch gekennzeichnet, daß in Formel I
X Schwefel oder Sauerstoff
R Fluor, Chlor, (C₁-C₃)-Alkyl oder Trifluormethyl,
m null, 1, 2 oder 3,
n 1 oder, falls m null, 1 oder 2 ist, auch 2,
R¹ (C₄-C₂₂)-Alkenyl, wobei gegebenenfalls eine CH₂-Gruppe durch Sauerstoff ersetzt sein kann,
R²,R³,R⁴ Wasserstoff, Hydroxy, Fluor, Chlor, (C₁-C₃)-Alkyl, (C₁-C₃)-Alkoxy, Trifluormethyl, wobei R², R³, R⁴ gleich oder verschieden sind, bedeuten.

7. Verbindungen gemäß Anspruch 1, dadurch gekennzeichnet, daß in Formel I
X Schwefel
R Fluor, Chlor, Methyl oder Trifluormethyl,
m null, 1, 2 oder 3,
n 1 oder, falls m null, 1 oder 2 ist, auch 2,
R¹ (C₄-C₂₂)-Alkenyl, wobei gegebenenfalls eine CH₂-Gruppe durch Sauerstoff ersetzt sein kann,
R²,R³,R⁴ Wasserstoff, Hydroxy, Fluor, Chlor, Methyl, Methoxy, Trifluormethyl, wobei R², R³, R⁴ gleich oder verschieden sind , bedeuten.

## Patentansprüche (Patentansprüche für folgende(n) Vertragsstaat(en): ES)

1. Verfahren zur Herstellung von substituierten 3-Thia- bzw. 3-Oxa-alkyl-Flavonen der Formel I, 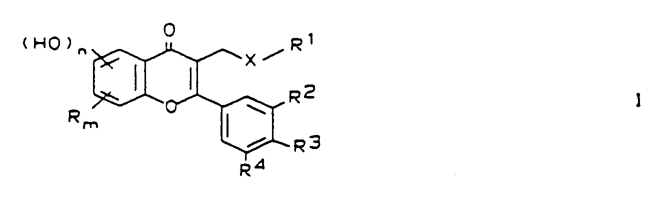 in der bedeuten:
X Schwefel oder Sauerstoff
R Halogen, (C₁-C₄)-Alkyl oder Trifluor-methyl,
m null, 1, 2 oder 3,
n 1 oder, falls m = null, 1 oder 2 ist, auch 2,
R¹ (C₃-C₂₅)-Alkenyl, wobei gegebenenfalls eine CH₂-Gruppe durch Sauerstoff ersetzt ist, oder (C₃-C₂₅)-Alkenyl, das mit Cyclohexenyl, das seinerseits 1 - 3 Methylgruppen enthält, substituiert ist,
R²,R³,R⁴ Wasserstoff, Hydroxy, Halogen, (C₁-C₄)-Alkyl, (C₁-C₄)-Alkoxy, Trifluormethyl, wobei R², R³, R⁴ gleich oder verschieden sind,
dadurch gekennzeichnet, daß man
a) ein Phenol der Formel II 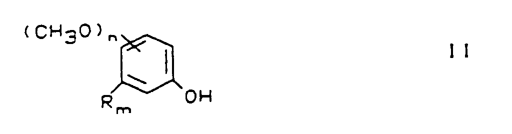 worin m, n und R die zur Formel I angegebene Bedeutung haben,
durch Acylierung in die Verbindungen der Formel III überführt 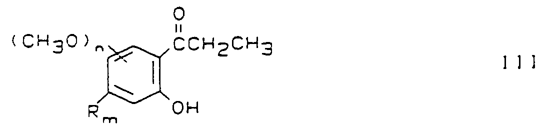 worin m, n und R die zur Formel I angegebene Bedeutung haben,
b) eine Verbindung der Formel III an der freien phenolischen Hydroxylgruppe mit einem Säurehalogenid der Formel IV, 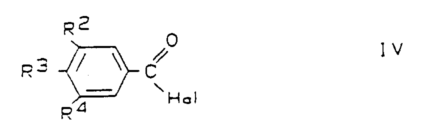 worin R², R³, R⁴ die zur Formel I angegebene Bedeutung haben und Hal gleich Brom oder Chlor ist, acyliert zu einer Verbindung der Formel V 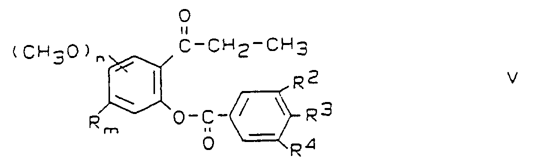 worin m, n, R und R², R³ R⁴ die zur Formel I angegebene Bedeutung haben,
c) eine Verbindung der Formel V cyclisiert zu einer Verbindung der Formel VI 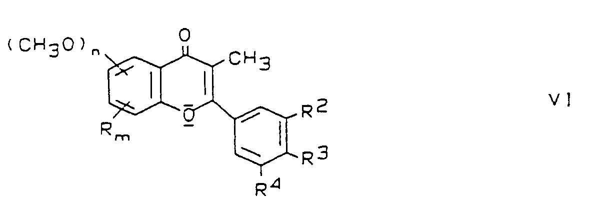 worin m, n, R und R², R³, R⁴ die zur Formel I angegebene Bedeutung haben,
d) in einer Verbindung der Formel VI die Methoxygruppen abspaltet, zu einer Verbindung der Formel VII 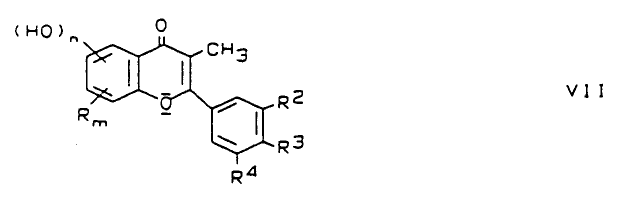 worin m, n und R sowie R², R³, R⁴ die zur Formel I angegebene Bedeutung haben,
e) eine Verbindung der Formel VII mit einem Acylierungsmittel zu einer Verbindung der Formel VIII umsetzt, 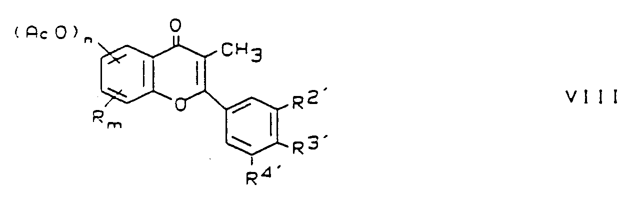 worin m, n und R die zur Formel I angegebene Bedeutung haben, und R^{2'}, R^{3'}, R^{4'} Wasserstoff, Halogen, (C₁-C₄)-Alkyl, (C₁-C₄)-Alkoxy, Trifluormethyl oder Acyloxy sind, wobei R^{2'}, R^{3'}, R^{4'} gleich oder verschieden sind,
f) eine Verbindung der Formel VIII selektiv halogeniert zu einer Verbindung der Formel IX 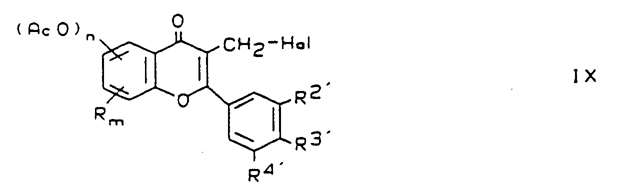 worin m, n und R die zur Formel I, R^{2'}, R^{3'}, R^{4'} die zur Formel VIII angegebene Bedeutung haben und Hal gleich Chlor, Brom oder Jod ist,
g) eine Verbindung der Formel IX umsetzt mit einer Verbindung der Formel X
H-X-R¹ X
worin X und R¹ die zur Formel I angegebene Bedeutung haben,
zu einer Verbindung der Formel XI 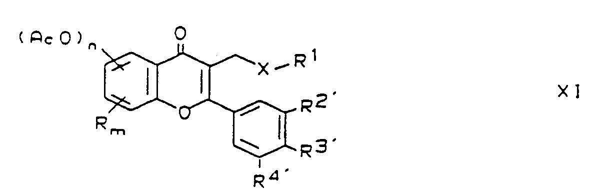 worin m, n, X, R und R¹ die zur Formel I und R^{2'}, R^{3'}, R^{4'} die zur Formel VIII angegebene Bedeutung haben,
h) in einer Verbindung der allgemeinen Formel XI die Acylschutzgruppen abspaltet zu einer Verbindung der
allgemeinen Formel I.

2. Verfahren zur Herstellung eines pharmazeutischen Präparates, dadurch gekennzeichnet, daß man eine Verbindung erhältlich gemäß Anspruch 1, in eine geeignete Darreichungsform überführt.

3. Verfahren gemäß Anspruch 1, dadurch gekennzeichnet, daß man eine Verbindung der Formel IX 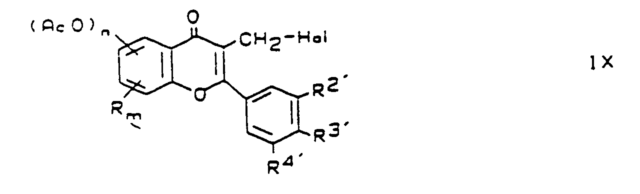 worin m, n und R die zur Formel I in Anspruch 1 und R^{2'}, R^{3'}, R^{4'} die zu Formel VIII in Anspruch 1 (e) angegebene Bedeutung haben , isoliert.

4. Verfahren gemäß Anspruch 1, dadurch gekennzeichnet, daß man eine Verbindung der Formel XI 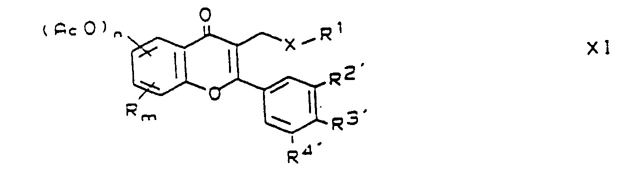 worin m, n und R die zur Formel I in Anspruch 1 und R^{2'}, R^{3'}, R^{4'} die zu Formel VIII in Anspruch 1 (e) angegebene Bedeutung haben , isoliert.

5. Verfahren gemäß Anspruch 1, dadurch gekennzeichnet, daß man eine Verbindung der Formel I, worin
X Schwefel oder Sauerstoff
R Fluor, Chlor, (C₁-C₃)-Alkyl oder Trifluormethyl,
m null, 1, 2 oder 3,
n 1 oder, falls m null, 1 oder 2 ist, auch 2,
R¹ (C₄-C₂₂)-Alkenyl, wobei gegebenenfalls eine CH₂-Gruppe durch Sauerstoff ersetzt sein kann,
R²,R³,R⁴ Wasserstoff, Hydroxy, Fluor, Chlor, (C₁-C₃)-Alkyl, (C₁-C₃)-Alkoxy, Trifluormethyl, wobei R², R³, R⁴ gleich oder verschieden sind , bedeuten, herstellt.

6. Verfahren gemäß Anspruch 1, dadurch gekennzeichnet, daß man eine Verbindung der Formel I, worin
X Schwefel
R Fluor, Chlor, Methyl oder Trifluormethyl,
m null, 1, 2 oder 3,
n 1 oder, falls m null, 1 oder 2 ist, auch 2,
R¹ (C₄-C₂₂)-Alkenyl, wobei gegebenenfalls eine CH₂-Gruppe durch Sauerstoff ersetzt sein kann,
R²,R³,R⁴ Wasserstoff, Hydroxy, Fluor, Chlor, Methyl, Methoxy, Trifluormethyl, wobei R², R³, R⁴ gleich oder verschieden sind , bedeuten, herstellt.

## Patentansprüche (Patentansprüche für folgende(n) Vertragsstaat(en): GR)

1. Verfahren zur Herstellung von substituierten 3-Thia- bzw. 3-Oxa-alkyl-Flavonen der Formel I, 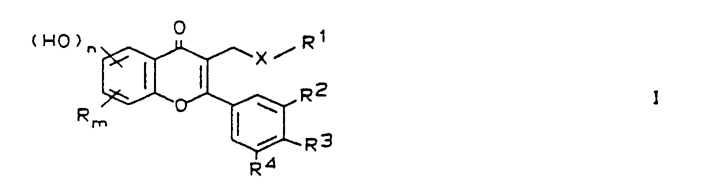 in der bedeuten:
X Schwefel oder Sauerstoff
R Halogen, (C₁-C₄)-Alkyl oder Trifluor-methyl,
m null, 1, 2 oder 3,
n 1 oder, falls m = null, 1 oder 2 ist, auch 2,
R¹ (C₃-C₂₅)-Alkenyl, wobei gegebenenfalls eine CH₂-Gruppe durch Sauerstoff ersetzt ist, oder (C₃-C₂₅)-Alkenyl, das mit Cyclohexenyl, das seinerseits 1 - 3 Methylgruppen enthält, substituiert ist,
R²,R³,R⁴ Wasserstoff, Hydroxy, Halogen, (C₁-C₄)-Alkyl, (C₁-C₄)-Alkoxy, Trifluormethyl, wobei R², R³, R⁴ gleich oder verschieden sind,
dadurch gekennzeichnet, daß man
a) ein Phenol der Formel II 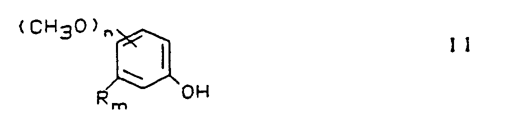 worin m, n und R die zur Formel I angegebene Bedeutung haben,
durch Acylierung in die Verbindungen der Formel III überführt 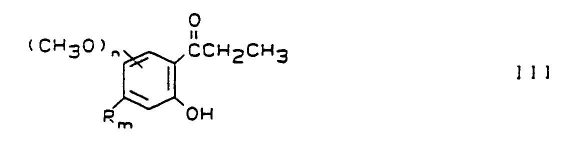 worin m, n und R die zur Formel I angegebene Bedeutung haben,
b) eine Verbindung der Formel III an der freien phenolischen Hydroxylgruppe mit einem Säurehalogenid der Formel IV, 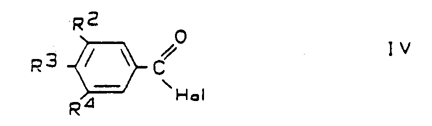 worin R², R³, R⁴ die zur Formel I angegebene Bedeutung haben und Hal gleich Brom oder Chlor ist, acyliert zu einer Verbindung der Formel V 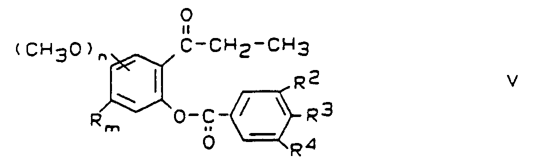 worin m, n, R und R², R³, R⁴ die zur Formel I angegebene Bedeutung haben,
c) eine Verbindung der Formel V cyclisiert zu einer Verbindung der Formel VI 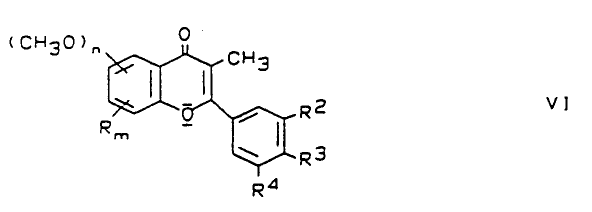 worin m, n, R und R², R³, R⁴ die zur Formel I angegebene Bedeutung haben,
d) in einer Verbindung der Formel VI die Methoxygruppen abspaltet, zu einer Verbindung der Formel VII 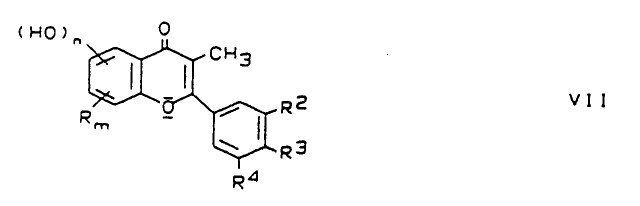 worin m, n und R sowie R², R³, R⁴ die zur Formel I angegebene Bedeutung haben,
e) eine Verbindung der Formel VII mit einem Acylierungsmittel zu einer Verbindung der Formel VIII umsetzt, 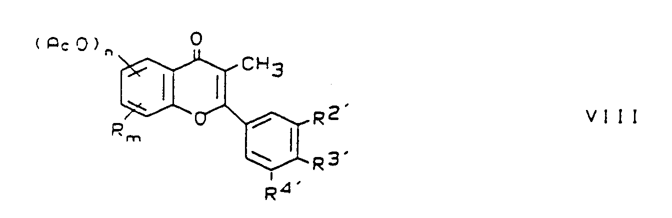 worin m, n und R die zur Formel I angegebene Bedeutung haben, und R^{2'}, R^{3'}, R^{4'} Wasserstoff, Halogen, (C₁-C₄)-Alkyl, (C₁-C₄)-Alkoxy, Trifluormethyl oder Acyloxy sind, wobei R^{2'}, R^{3'}, R^{4'} gleich oder verschieden sind,
f) eine Verbindung der Formel VIII selektiv halogeniert zu einer Verbindung der Formel IX 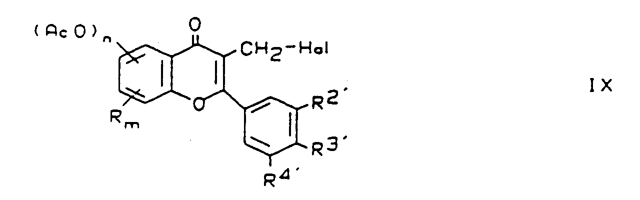 worin m, n und R die zur Formel I, R^{2'}, R^{3'}, R^{4'} die zur Formel VIII angegebene Bedeutung haben und Hal gleich Chlor, Brom oder Jod ist,
g) eine Verbindung der Formel IX umsetzt mit einer Verbindung der Formel X
H-X-R¹ X
worin X und R¹ die zur Formel I angegebene Bedeutung haben,
zu einer Verbindung der Formel XI 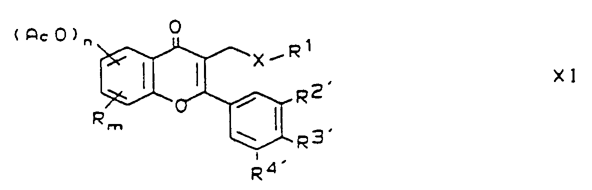 worin m, n, X, R und R¹ die zur Formel I und R^{2'}, R^{3'}, R^{4'} die zur Formel VIII angegebene Bedeutung haben,
h) in einer Verbindung der allgemeinen Formel XI die Acylschutzgruppen abspaltet zu einer Verbindung der allgemeinen Formel I.

2. Verfahren zur Herstellung eines pharmazeutischen Präparates, dadurch gekennzeichnet, daß man eine Verbindung erhältlich gemäß Anspruch 1, in eine geeignete Darreichungsform überführt.

3. Verbindungen der Formel IX 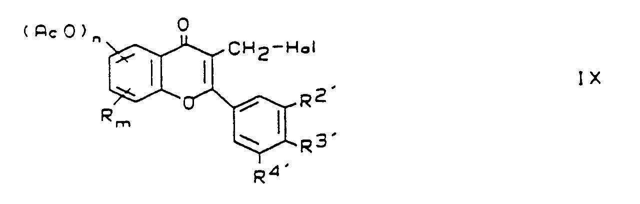 worin m, n und R die zur Formel I in Anspruch 1 und R^{2'}, R^{3'}, R^{4'} die zu Formel VIII in Anspruch 1 (e) angegebene Bedeutung haben.

4. Verbindungen der Formel XI 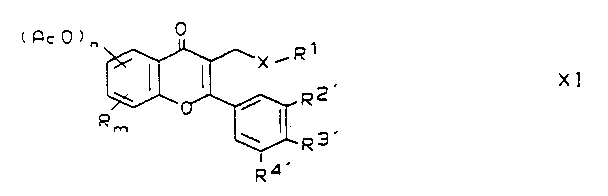 worin m, n, X, R und R¹ die zur Formel I in Anspruch 1 und R^{2'}, R^{3'}, R^{4'} die zur Formel VIII in Anspruch 1 (e) angegebene Bedeutung haben.

5. Verfahren gemäß Anspruch 1, dadurch gekennzeichnet, daß man eine Verbindung der Formel I, worin
X Schwefel oder Sauerstoff
R Fluor, Chlor, (C₁-C₃)-Alkyl oder Trifluormethyl,
m null, 1, 2 oder 3,
n 1 oder, falls m null, 1 oder 2 ist, auch 2,
R¹ (C₄-C₂₂)-Alkenyl, wobei gegebenenfalls eine CH₂-Gruppe durch Sauerstoff ersetzt sein kann,
R²,R³,R⁴ Wasserstoff, Hydroxy, Fluor, Chlor, (C₁-C₃)-Alkyl, (C₁-C₃)-Alkoxy, Trifluormethyl, wobei R², R³, R⁴ gleich oder verschieden sind, bedeuten, herstellt.

6. Verfahren gemäß Anspruch 1, dadurch gekennzeichnet, daß man eine Verbindung der Formel I, worin
X Schwefel
R Fluor, Chlor, Methyl oder Trifluormethyl,
m null, 1, 2 oder 3,
n 1 oder, falls m null, 1 oder 2 ist, auch 2,
R¹ (C₄-C₂₂)-Alkenyl, wobei gegebenenfalls eine CH₂-Gruppe durch Sauerstoff ersetzt sein kann,
R²,R³,R⁴ Wasserstoff, Hydroxy, Fluor, Chlor, Methyl, Methoxy, Trifluormethyl, wobei R², R³, R⁴ gleich oder verschieden sind , bedeuten, herstellt.

## Claims (Claims for the following Contracting State(s): AT, BE, CH, DE, DK, FR, GB, IT, LI, LU, NL, SE)

1. A substituted 3-thia- or 3-oxa-alkylflavone of formula I 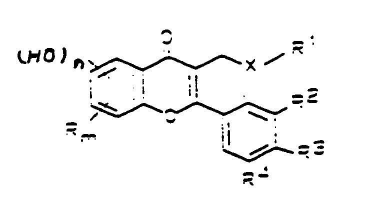 in which:
X is sulfur or oxygen,
R is halogen, (C₁-C₄)-alkyl or trifluoromethyl,
m is zero, 1, 2 or 3,
n is 1 or, if m = zero, 1 or 2, also 2,
R¹ is (C₃-C₂₅)-alkenyl, one CH₂ group optionally being replaced by oxygen, or is (C₃-C₂₅)-alkenyl which is substituted by cyclohexenyl, which in turn contains 1 - 3 methyl groups, and
R², R³ and R⁴ are hydrogen, hydroxyl, halogen, (C₁-C₄)-alkyl, (C₁-C₄)-alkoxy or trifluoromethyl, R², R³ and R⁴ being identical or different.

2. A process for the preparation of compounds of formula I, which comprises
a) converting a phenol of formula II 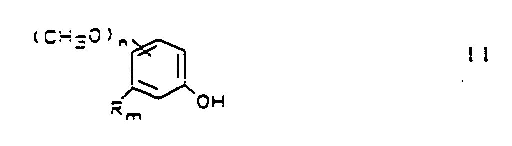 in which m, n and R have the meaning indicated for formula I,
by acylation into the compounds of formula III 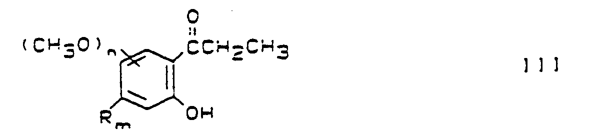 in which m, n and R have the meaning indicated for formula I,
b) acylating a compound of formula III on the free phenolic hydroxyl group using an acid halide of formula IV 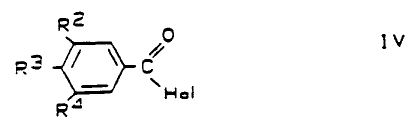 in which R², R³ and R⁴ have the meaning indicated for formula I, and Hal is bromine or chlorine, to give a compound of formula V 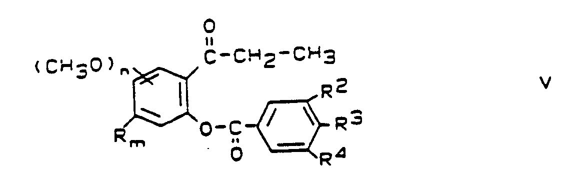 in which m, n, R and R², R³ and R⁴ have the meaning indicated for formula I,
c) cyclizing a compound of formula V to give a compound of formula VI 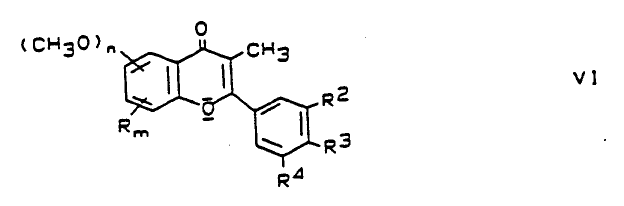 in which m, n, R and R², R³ and R⁴ have the meaning indicated for formula I,
d) splitting off the methoxy groups from a compound of formula VI, to give a compound of formula VII 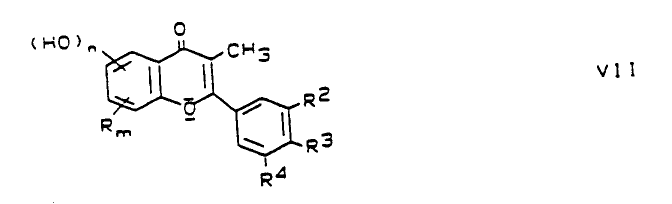 in which m, n and R as well as R², R³ and R⁴ have the meaning indicated for formula I,
e) reacting a compound of formula VII with an acylating agent to give a compound of formula VIII 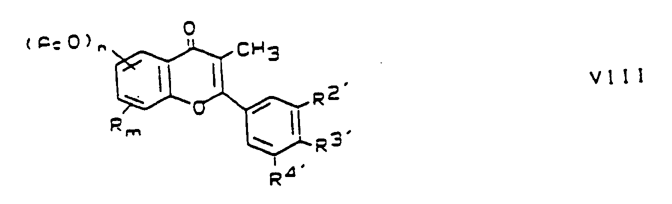 in which m, n and R have the meaning indicated for formula I, and R^{2'}, R^{3'} and R^{4'} are hydrogen, halogen, (C₁-C₄)-alkyl, (C₁-C₄)-alkoxy, trifluoromethyl or acyloxy, R^{2'}, R^{3'} and R^{4'} being identical or different,
f) selectively halogenating a compound of formula VIII to give a compound of formula IX 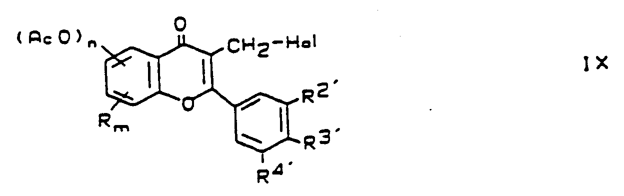 in which m, n and R have the meaning indicated for formula I, R^{2'}, R^{3'} and R^{4'} have the meaning indicated for formula VIII, and Hal is chlorine, bromine or iodine,
g) reacting a compound of formula IX with a compound of formula X
H-X-R¹ X
in which X and R¹ have the meaning indicated for formula I,
to give a compound of formula XI 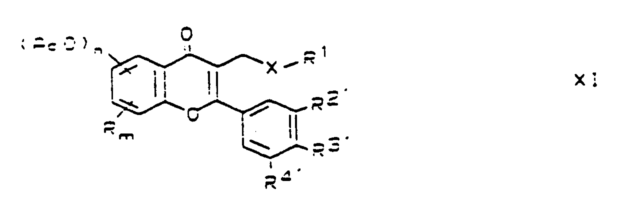 in which m, n, X, R and R¹ have the meaning indicated for formula I, and R^{2'}, R^{3'} and R^{4'} have the meaning indicated for formula VIII, and
h) splitting off the acyl protective groups from a compound of formula XI, to give a compound of formula I.

3. A pharmaceutical preparation which contains a compound as claimed in claim 1.

4. A compound of formula IX 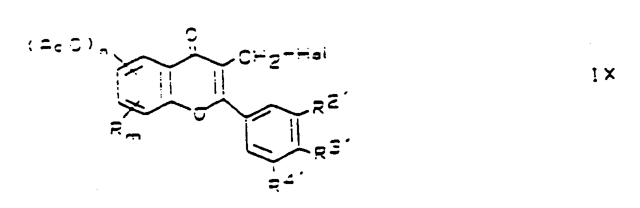 in which m, n and R have the meaning indicated for formula I in claim 1 and R^{2'}, R^{3'} and R^{4'} have the meaning indicated for formula VIII in claim 2(e).

5. A compound of formula XI 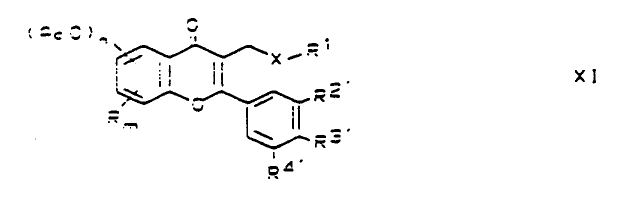 in which m, n, X, R and R¹ have the meaning indicated for formula I in claim 1 and R^{2'}, R^{3'} and R^{4'} have the meaning indicated for formula VIII in claim 2(e).

6. A compound as claimed in claim 1, wherein, in formula I,
X is sulfur or oxygen,
R is fluorine, chlorine, (C₁-C₃)-alkyl or trifluoromethyl,
m is zero, 1, 2 or 3,
n is 1 or, if m is zero, 1 or 2, also 2,
R¹ is (C₄-C₂₂)-alkenyl, it being possible for one CH₂ group optionally to be replaced by oxygen, and
R², R³ and R⁴ are hydrogen, hydroxyl, fluorine, chlorine, (C₁-C₃)-alkyl, (C₁-C₃)-alkoxy or trifluoromethyl, R², R³ and R⁴ being identical or different.

7. A compound as claimed in claim 1, wherein, in formula I,
X is sulfur,
R is fluorine, chlorine, methyl or trifluoromethyl,
m is zero, 1, 2 or 3,
n is 1 or, if m is zero, 1 or 2, also 2,
R¹ is (C₄-C₂₂)-alkenyl, it being possible for one CH₂ group optionally to be replaced by oxygen, and
R², R³ and R⁴ are hydrogen, hydroxyl, fluorine, chlorine, methyl, methoxy or trifluoromethyl, R², R³ and R⁴ being identical or different.

## Claims (Claims for the following Contracting State(s): ES)

1. A process for the preparation of substituted 3-thia-or 3-oxa-alkylflavones of formula I 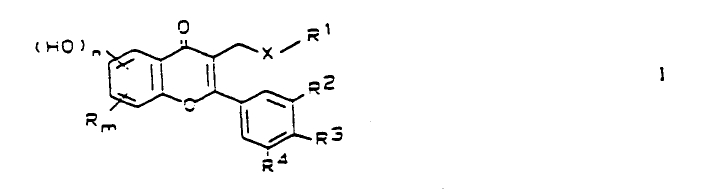 in which:
X is sulfur or oxygen,
R is halogen, (C₁-C₄)-alkyl or trifluoromethyl,
m is zero, 1, 2 or 3,
n is 1 or, if m = zero, 1 or 2, also 2,
R¹ is (C₃-C₂₅)-alkenyl, one CH₂ group optionally being replaced by oxygen, or is (C₃-C₂₅)-alkenyl which is substituted by cyclohexenyl, which in turn contains 1 - 3 methyl groups, and
R², R³ and R⁴ are hydrogen, hydroxyl, halogen, (C₁-C₄)-alkyl, (C₁-C₄)-alkoxy or trifluoromethyl, R², R³ and R⁴ being identical or different,
which comprises
a) converting a phenol of formula II 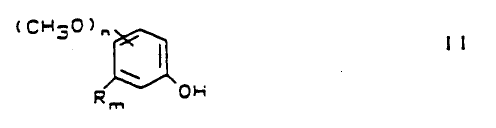 in which m, n and R have the meaning indicated for formula I,
by acylation into the compounds of formula III 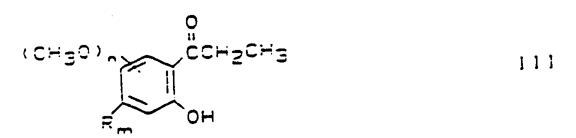 in which m, n and R have the meaning indicated for formula I,
b) acylating a compound of formula III on the free phenolic hydroxyl group using an acid halide of formula IV 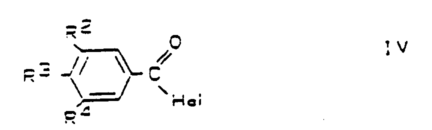 in which R², R³ and R⁴ have the meaning indicated for formula I, and Hal is bromine or chlorine, to give a compound of formula V 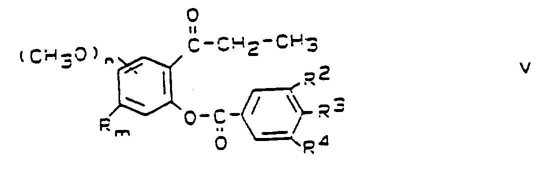 in which m, n, R and R², R³ and R⁴ have the meaning indicated for formula I,
c) cyclizing a compound of formula V to give a compound of formula VI 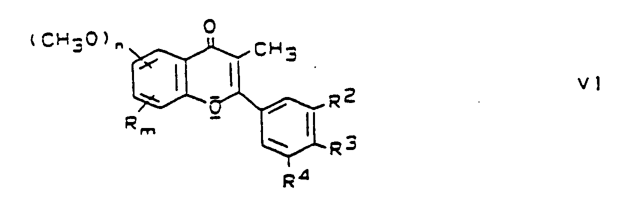 in which m, n, R and R², R³ and R⁴ have the meaning indicated for formula I,
d) splitting off the methoxy groups from a compound of formula VI, to give a compound of formula VII 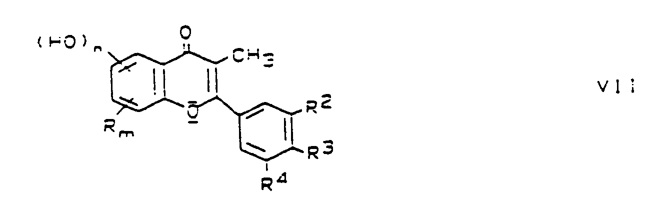 in which m, n and R as well as R², R³ and R⁴ have the meaning indicated for formula I,
e) reacting a compound of formula VII with an acylating agent to give a compound of formula VIII 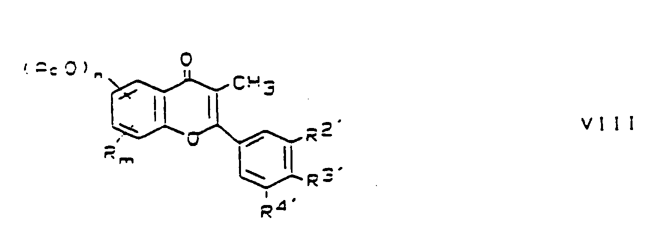 in which m, n and R have the meaning indicated for formula I, and R^{2'}, R^{3'} and R^{4'} are hydrogen, halogen, (C₁-C₄)-alkyl, (C₁-C₄)-alkoxy, trifluoromethyl or acyloxy, R^{2'}, R^{3'} and R^{4'} being identical or different,
f) selectively halogenating a compound of formula VIII to give a compound of formula IX 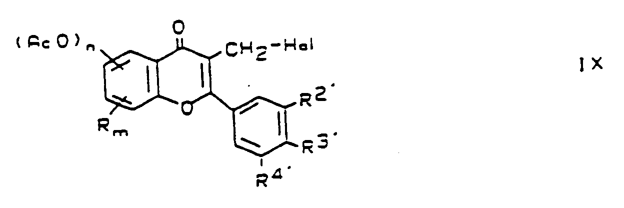 in which m, n and R have the meaning indicated for formula I, R^{²'}, R^{3'} and R^{4'} have the meaning indicated for formula VIII, and Hal is chlorine, bromine or iodine,
g) reacting a compound of formula IX with a compound of formula X
H-X-R¹ X
in which X and R¹ have the meaning indicated for formula I,
to give a compound of formula XI 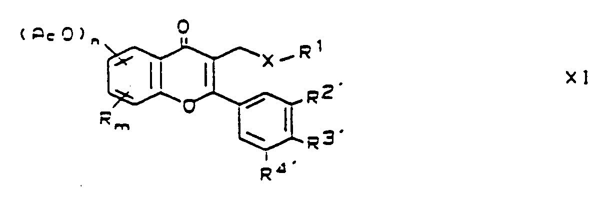 in which m, n, X, R and R¹ have the meaning indicated for formula I, and R^{2'}, R^{3'} and R^{4'} have the meaning indicated for formula VIII, and
h) splitting off the acyl protective groups from a compound of formula XI, to give a compound of formula I.

2. A process for the production of a pharmaceutical preparation, which comprises converting a compound obtainable as claimed in claim 1 into a suitable dosage form.

3. The process as claimed in claim 1, wherein a compound of formula IX 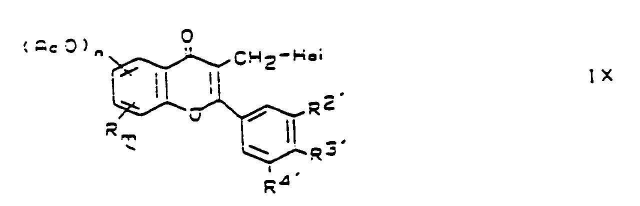 in which m, n and R have the meaning indicated for formula I in claim 1 and R^{2'}, R^{3'} and R^{4'} have the meaning indicated for formula VIII in claim 1(e), is isolated.

4. The process as claimed in claim 1, wherein a compound of formula XI 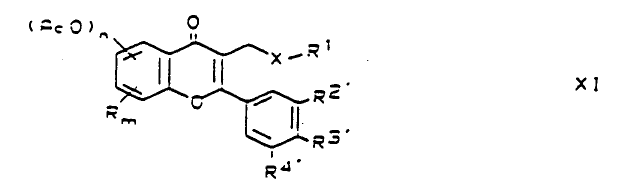 in which m, n, X, R and R¹ have the meaning indicated for formula I in claim 1 and R^{2'}, R^{3'} and R^{4'} have the meaning indicated for formula VIII in claim 1(e), is isolated.

5. The process as claimed in claim 1, wherein a compound of formula I in which
X is sulfur or oxygen,
R is fluorine, chlorine, (C₁-C₃)-alkyl or trifluoromethyl,
m is zero, 1, 2 or 3,
n is 1 or, if m is zero, 1 or 2, also 2,
R¹ is (C₄-C₂₂)-alkenyl, it being possible for one CH₂ group optionally to be replaced by oxygen, and
R², R³ and R⁴ are hydrogen, hydroxyl, fluorine, chlorine, (C₁-C₃)-alkyl, (C₁-C₃)-alkoxy or trifluoromethyl, R², R³ and R⁴ being identical or different, is prepared.

6. The process as claimed in claim 1, wherein a compound of formula I in which
X is sulfur,
R is fluorine, chlorine, methyl or trifluoromethyl,
m is zero, 1, 2 or 3,
n is 1 or, if m is zero, 1 or 2, also 2,
R¹ is (C₄-C₂₂)-alkenyl, it being possible for one CH₂ group optionally to be replaced by oxygen, and
R², R³ and R⁴ are hydrogen, hydroxyl, fluorine, chlorine, methyl, methoxy or trifluoromethyl, R², R³ and R⁴ being identical or different, is prepared.

## Claims (Claims for the following Contracting State(s): GR)

1. A process for the preparation of substituted 3-thia-or 3-oxa-alkylflavones of formula I 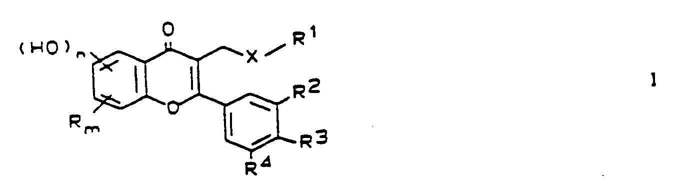 in which:
X is sulfur or oxygen,
R is halogen, (C₁-C₄)-alkyl or trifluoromethyl,
m is zero, 1, 2 or 3,
n is 1 or, if m = zero, 1 or 2, also 2,
R¹ is (C₃-C₂₅)-alkenyl, one CH₂ group optionally being replaced by oxygen, or is (C₃-C₂₅)-alkenyl which is substituted by cyclohoxenyl, which in turn contains 1 - 3 methyl groups, and
R², R³ and R⁴ are hydrogen, hydroxyl, halogen, (C₁-C₄)-alkyl, (C₁-C₄)-alkoxy or trifluoromethyl, R², R³ and R⁴ being identical or different,
which comprises
a) converting a phenol of formula II 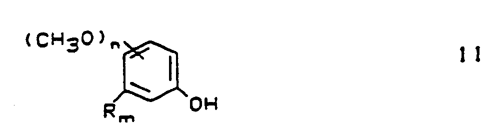 in which m, n and R have the meaning indicated for formula I,
by acylation into the compounds of formula III 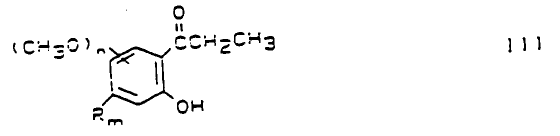 in which m, n and R have the meaning indicated for formula I,
b) acylating a compound of formula III on the free phenolic hydroxyl group using an acid halide of formula IV 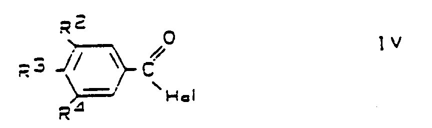 in which R², R³ and R⁴ have the meaning indicated for formula I, and Hal is bromine or chlorine, to give a compound of formula V 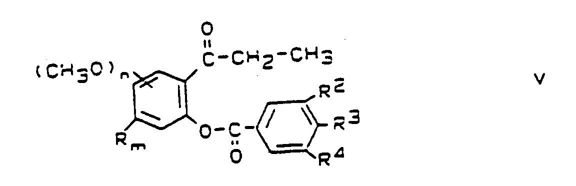 in which m, n, R and R², R³ and R⁴ have the meaning indicated for formula I,
c) cyclizing a compound of formula V to give a compound of formula VI 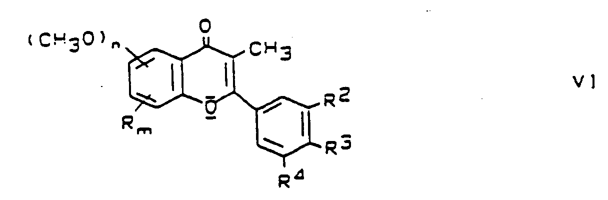 in which m, n, R and R², R³ and R⁴ have the meaning indicated for formula I,
d) splitting off the methoxy groups from a compound of formula VI, to give a compound of formula VII 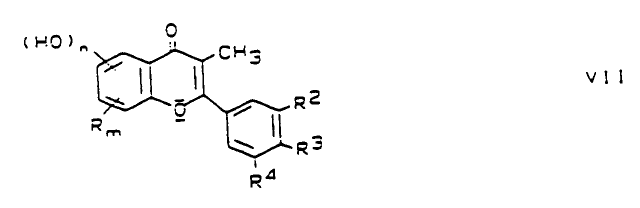 in which m, n and R as well as R², R³ and R⁴ have the meaning indicated for formula I,
e) reacting a compound of formula VII with an acylating agent to give a compound of formula VIII 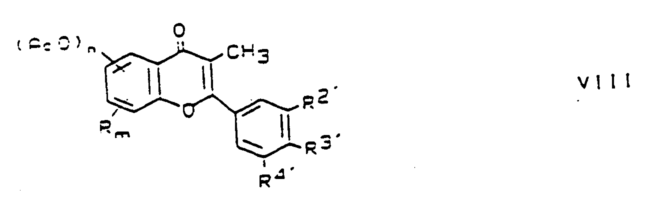 in which m, n and R have the meaning indicated for formula I, and R^{2'}, R^{3'} and R^{4'} are hydrogen, halogen, (C₁-C₄)-alkyl, (C₁-C₄)-alkoxy, trifluoromethyl or acyloxy, R^{2'}, R^{3'} and R^{4'} being identical or different,
f) selectively halogenating a compound of formula VIII to give a compound of formula IX 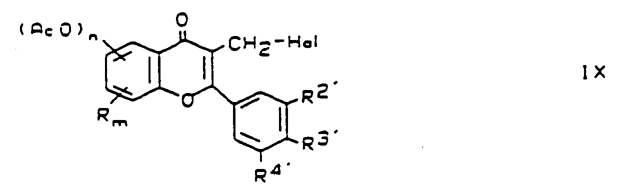 in which m, n and R have the meaning indicated for formula I, R^{2'}, R^{3'} and R^{4'} have the meaning indicated for formula VIII, and Hal is chlorine, bromine or iodine,
g) reacting a compound of formula IX with a compound of formula X
H-X-R¹ X
in which X and R¹ have the meaning indicated for formula I,
to give a compound of formula XI 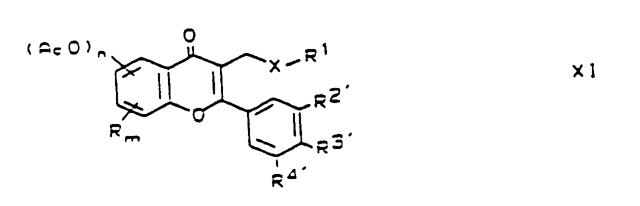 in which m, n, X, R and R¹ have the meaning indicated for formula I, and R^{2'}, R^{3'} and R^{4'} have the meaning indicated for formula VIII, and
h) splitting off the acyl protective groups from a compound of formula XI, to give a compound of formula I.

2. A process for the production of a pharmaceutical preparation, which comprises converting a compound obtainable as claimed in claim 1 into a suitable dosage form.

3. A compound of formula IX 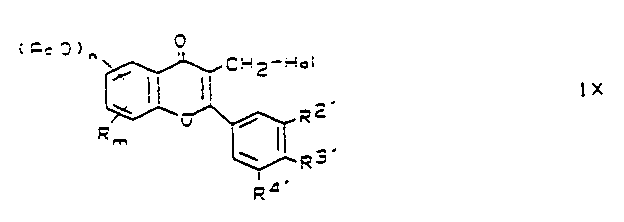 in which m, n and R have the meaning indicated for formula I in claim 1 and R^{2'}, R^{3'} and R^{4'} have the meaning indicated for formula VIII in claim 1(e).

4. A compound of formula XI 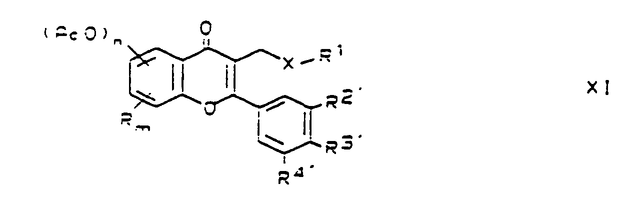 in which m, n, X, R and R¹ have the meaning indicated for formula I in claim 1 and R^{2'}, R^{3'} and R^{4'} have the meaning indicated for formula VIII in claim 1(e).

5. The process as claimed in claim 1, wherein a compound of formula I in which
X is sulfur or oxygen,
R is fluorine, chlorine, (C₁-C₃)-alkyl or trifluoromethyl,
m is zero, 1, 2 or 3,
n is 1 or, if m is zero, 1 or 2, also 2,
R¹ is (C₄-C₂₂)-alkenyl, it being possible for one CH₂ group optionally to be replaced by oxygen, and
R², R³ and R⁴ are hydrogen, hydroxyl, fluorine, chlorine, (C₁-C₃)-alkyl, (C₁-C₃)-alkoxy or trifluoromethyl, R², R³ and R⁴ being identical or different, is prepared.

6. The process as claimed in claim 1, wherein a compound of formula I in which
X is sulfur,
R is fluorine, chlorine, methyl or trifluoromethyl,
m is zero, 1, 2 or 3,
n is 1 or, if m is zero, 1 or 2, also 2,
R¹ is (C₄-C₂₂)-alkenyl, it being possible for one CH₂ group optionally to be replaced by oxygen, and
R², R³ and R⁴ are hydrogen, hydroxyl, fluorine, chlorine, methyl, methoxy or trifluoromethyl, R², R³ and R⁴ being identical or different, is prepared.

## Revendications (Revendications pour l'(les) Etat(s) contractant(s) suivant(s): AT, BE, CH, DE, DK, FR, GB, IT, LI, LU, NL, SE)

1. 3-thia- ou 3-oxa-alkylflavones substituées de formule I 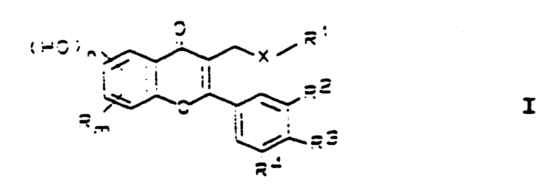 dans laquelle
X représente un atome de soufre ou d'oxygène,
R représente un atome d'halogène ou un groupe alkyle en C₁-C₄ ou trifluorométhyle;
m est zéro, 1, 2 ou 3,
n est 1 ou, lorsque m = zéro, 1 ou 2, également 2,
R¹ représente un radical alcényle en C₃-C₂₅, éventuellement un groupe CH₂ étant remplacé par un atome d'oxygène, ou un radical alcényle en C₃-C₂₅ qui est substitué par un groupe cyclohexényle qui, pour sa part, contient 1-3 groupes méthyle,
R², R³, R⁴ représentent un atome d'hydrogène ou d'halogène, ou un groupe hydroxy, alkyle en C₁-C₄, alcoxy en C₁-C₄, trifluorométhyle, R², R³, R⁴ étant identiques ou différents.

2. Procédé pour la préparation des composés de formule I, caractérisé en ce que
a) on convertit un phénol de formule II 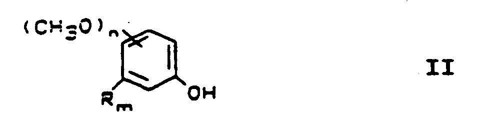 dans laquelle m, n et R ont les significations données à propos de la formule I,
par acylation, en les composés de formule III 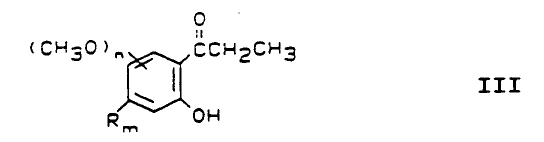 dans laquelle m, n et R ont les significations données à propos de la formule I,
b) on soumet un composé de formule III à une acylation sur le groupe hydroxy phénolique libre, avec un halogénure d'acide de formule IV 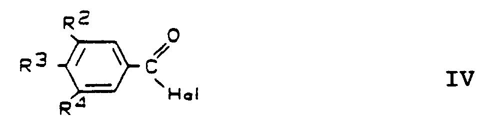 dans laquelle R², R³, R⁴ ont les significations données à propos de la formule I et Hal est un atome de chlore ou de brome,
pour aboutir à un composé de formule V 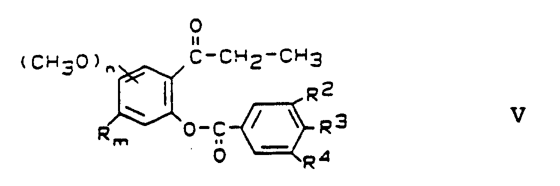 dans laquelle m, n, R et R², R³, R⁴ ont les significations données à propos de la formule I,
c) on cyclise un composé de formule V en un composé de formule VI 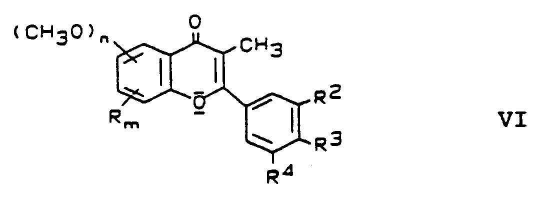 dans laquelle m, n, R et R², R³, R⁴ ont les significations données à propos de la formule I,
d) dans un composé de formule VI, on élimine les groupes méthoxy, pour aboutir à un composé de formule VII 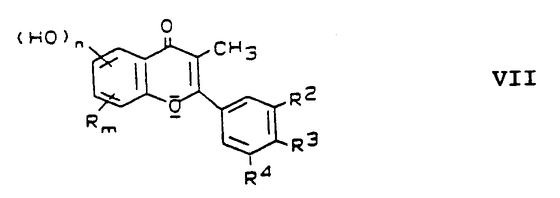 dans laquelle m, n et R, ainsi que R², R³, R⁴ ont les significations données à propos de la formule I,
e) on fait réagir un composé de formule VII avec un agent d'acylation, pour aboutir à un composé de formule VIII 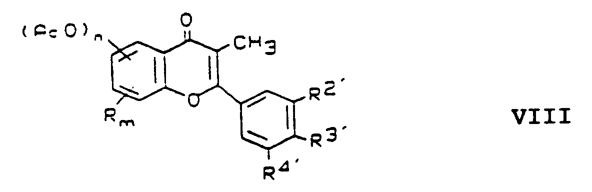 dans laquelle m, n et R ont les significations données à propos de la formule I, et R^{2'}, R^{3'}, R^{4'} représentent un atome d'hydrogène ou d'halogène, ou un groupe alkyle en C₁-C₄, alcoxy en C₁-C₄, trifluorométhyle ou acyloxy, R^{2'}, R^{3'}, R^{4'} étant identiques ou différents,
f) on soumet à une halogénation sélective un composé de formule VIII, pour aboutir à un composé de formule IX 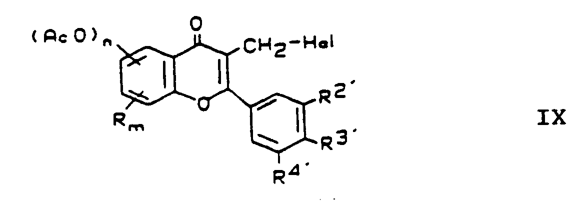 dans laquelle m, n et R ont les significations données à propos de la formule I, R^{2'}, R^{3'}, R^{4'} ont les significations données à propos de la formule VIII et Hal est un atome de chlore, brome ou iode,
g) on fait réagir un composé de formule IX avec un composé de formule X
H-X-R¹ X
dans laquelle X et R¹ ont les significations données à propos de la formule I,
pour aboutir à un composé de formule XI 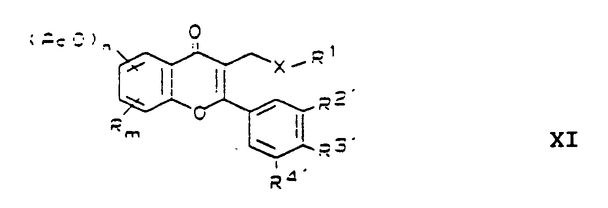 dans laquelle m, n, X, R et R¹ ont les significations données à propos de la formule I et R^{2'}, R^{3'}, R^{4'} ont les significations données à propos de la formule VIII,
h) dans un composé de formule générale XI, on élimine les groupes protecteurs acyle, pour aboutir à un composé de formule générale I.

3. Composition pharmaceutique, caractérisée en ce qu'elle contient un composé selon la revendication 1.

4. Composés de formule IX 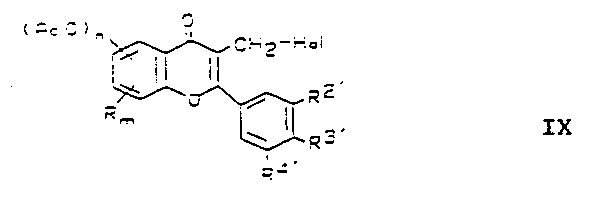 dans laquelle m, n et R ont les significations données à propos de la revendication 1 et R^{2'}, R^{3'}, R^{4'} ont les significations données à propos de la formule VIII dans la revendication 2 (e).

5. Composés de formule XI 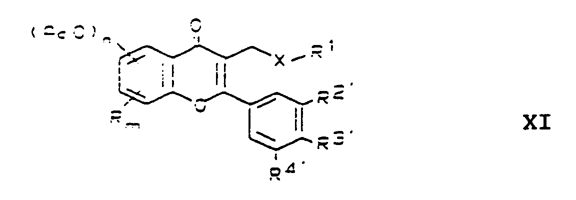 dans laquelle m, n, X, R et R¹ ont les significations données à propos de la formule I dans la revendication 1 et R^{2'}, R^{3'}, R^{4'} ont les significations données à propos de la formule VIII dans la revendication 2 (e).

6. Composés selon la revendication 1, caractérisés en ce que, dans la formule I,
X représente un atome de soufre ou d'oxygène,
R représente un atome de fluor ou de chlore, ou un groupe alkyle en C₁-C₃ ou trifluorométhyle,
m est zéro, 1, 2 ou 3,
n est 1 ou, lorsque m est zéro, 1 ou 2, également 2,
R¹ représente un groupe alcényle en C₄-C₂₂, éventuellement un groupe CH₂ pouvant être remplacé par un atome d'oxygène,
R², R³, R⁴ représentent un atome d'hydrogène, de fluor ou de chlore, ou un groupe hydroxy, alkyle en C₁-C₃, alcoxy en C₁-C₃ ou trifluorométhyle, R², R³, R⁴ étant identiques ou différents.

7. Composés selon la revendication 1, caractérisés en ce que, dans la formule I,
X représente un atome de soufre,
R représente un atome de fluor ou de chlore, ou le groupe méthyle ou trifluorométhyle,
m est zéro, 1, 2 ou 3,
n est 1 ou, lorsque m est zéro, 1 ou 2, également 2,
R¹ représente un groupe alcényle en C₄-C₂₂, éventuellement un groupe CH₂ pouvant être remplacé par un atome d'oxygène,
R², R³, R⁴ représentent un atome d'hydrogène, de fluor ou de chlore, ou le groupe hydroxy, méthyle, méthoxy ou trifluorométhyle, R², R³, R⁴ étant identiques ou différents.

## Revendications (Revendications pour l'(les) Etat(s) contractant(s) suivant(s): ES)

1. Procédé pour la préparation de 3-thia- ou 3-oxa-alkylflavones substituées de formule I 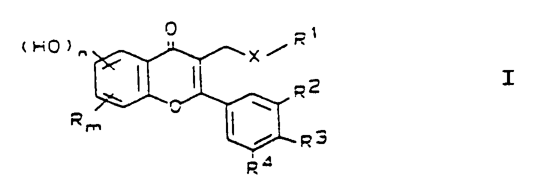 dans laquelle
X représente un atome de soufre ou d'oxygène,
R représente un atome d'halogène ou un groupe alkyle en C₁-C₄ ou trifluorométhyle;
m est zéro, 1, 2 ou 3,
n est 1 ou, lorsque m = zéro, 1 ou 2, également 2,
R¹ représente un radical alcényle en C₃-C₂₅, éventuellement un groupe CH₂ étant remplacé par un atome d'oxygène, ou un radical alcényle en C₃-C₂₅ qui est substitué par un groupe cyclohexényle qui, pour sa part, contient 1-3 groupes méthyle,
R², R³, R⁴ représentent un atome d'hydrogène ou d'halogène, ou un groupe hydroxy, alkyle en C₁-C₄, alcoxy en C₁-C₄, trifluorométhyle, R², R³, R⁴ étant identiques ou différents,
caractérisé en ce que
a) on convertit un phénol de formule II 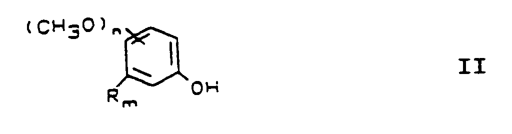 dans laquelle m, n et R ont les significations données à propos de la formule I,
par acylation, en les composés de formule III 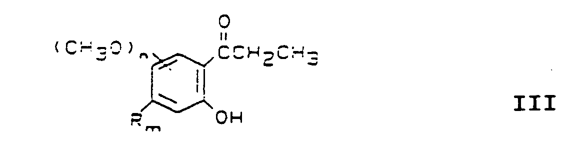 dans laquelle m, n et R ont les significations données à propos de la formule I,
b) on soumet un composé de formule III à une acylation sur le groupe hydroxy phénolique libre, avec un halogénure d'acide de formule IV 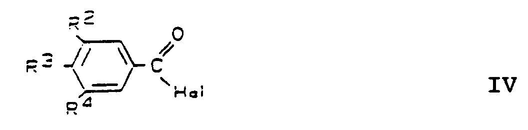 dans laquelle R², R³, R⁴ ont les significations données à propos de la formule I et Hal est un atome de chlore ou de brome,
pour aboutir à un composé de formule V 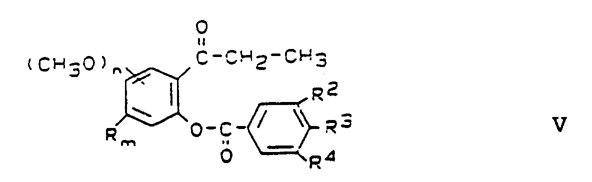 dans laquelle m, n, R et R², R³, R⁴ ont les significations données à propos de la formule I,
c) on cyclise un composé de formule V en un composé de formule VI 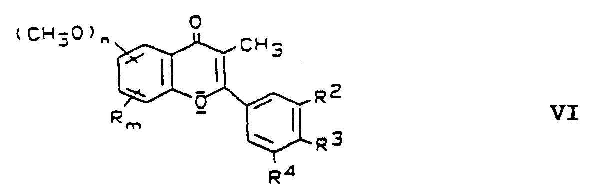 dans laquelle m, n, R et R², R³, R⁴ ont les significations données à propos de la formule I,
d) dans un composé de formule VI, on élimine les groupes méthoxy, pour aboutir à un composé de formule VII 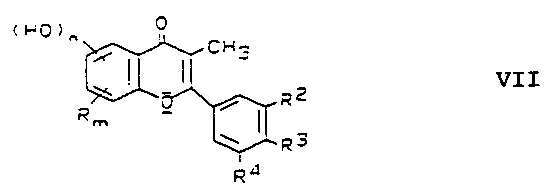 dans laquelle m, n et R, ainsi que R², R³, R⁴ ont les significations données à propos de la formule I,
e) on fait réagir un composé de formule VII avec un agent d'acylation, pour aboutir à un composé de formule VIII 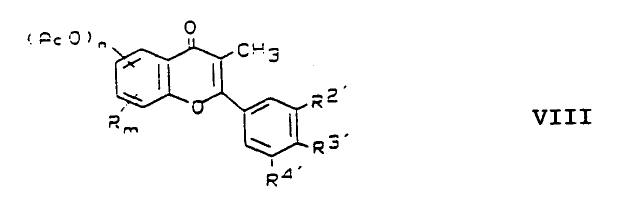 dans laquelle m, n et R ont les significations données à propos de la formule I, et R^{2'}, R^{3'}, R^{4'} représentent un atome d'hydrogène ou d'halogène, ou un groupe alkyle en C₁-C₄, alcoxy en C₁-C₄, trifluorométhyle ou acyloxy, R^{2'}, R^{3'}, R^{4'} étant identiques ou différents,
f) on soumet à une halogénation sélective un composé de formule VIII, pour aboutir à un composé de formule IX 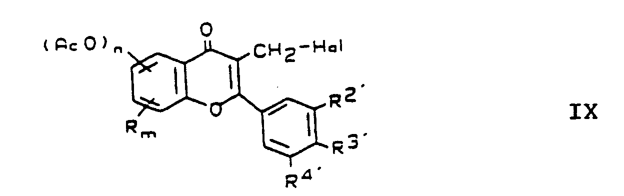 dans laquelle m, n et R ont les significations données à propos de la formule I, R^{2'}, R^{3'}, R^{4'} ont les significations données à propos de la formule VIII et Hal est un atome de chlore, brome ou iode,
g) on fait réagir un composé de formule IX avec un composé de formule X
H-X-R¹ X
dans laquelle X et R¹ ont les significations données à propos de la formule I,
pour aboutir à un composé de formule XI 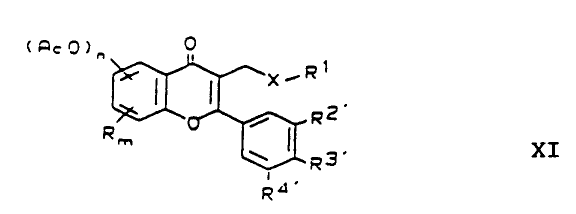 dans laquelle m, n, X, R et R¹ ont les significations données à propos de la formule I et R^{2'}, R^{3'}, R^{4'} ont les significations données à propos de la formule VIII,
h) dans un composé de formule générale XI, on élimine les groupes protecteurs acyle, pour aboutir à un composé de formule générale I.

2. Procédé pour la préparation d'une composition pharmaceutique, caractérisée en ce que l'on met sous une forme d'administration appropriée un composé préparable selon la revendication 1.

3. Procédé selon la revendication 1, caractérisé en ce que l'on isole un composé de formule IX 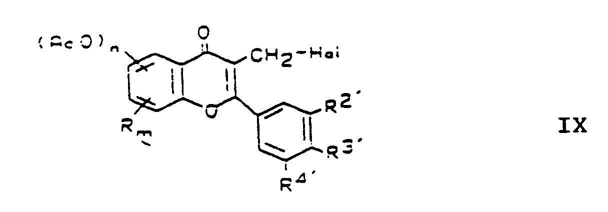 dans laquelle m, n et R ont les significations données à propos de la formule I dans la revendication 1 et R^{2'}, R^{3'}, R^{4'} ont les significations données à propos de la formule VIII dans la revendication 1 (e).

4. Procédé selon la revendication 1, caractérisé en ce que l'on isole des composés de formule XI 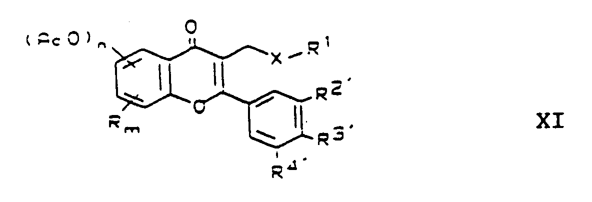 dans laquelle m, n, X, R et R¹ ont les significations données à propos de la formule I dans la revendication 1 et R^{2'}, R^{3'}, R^{4'} ont les significations données à propos de la formule VIII dans la revendication 1 (e).

5. Procédé selon la revendication 1, caractérisé en ce que l'on prépare un composé de formule I dans lequel,
X représente un atome de soufre ou d'oxygène,
R représente un atome de fluor ou de chlore, ou un groupe alkyle en C₁-C₃ ou trifluorométhyle,
m est zéro, 1, 2 ou 3,
n est 1 ou, lorsque m est zéro, 1 ou 2, également 2,
R¹ représente un groupe alcényle en C₄-C₂₂, éventuellement un groupe CH₂ pouvant être remplacé par un atome d'oxygène,
R², R³, R⁴ représentent un atome d'hydrogène, de fluor ou de chlore, ou un groupe hydroxy, alkyle en C₁-C₃, alcoxy en C₁-C₃ ou trifluorométhyle, R², R³, R⁴ étant identiques ou différents.

6. Procédé selon la revendication 1, caractérisé en ce que l'on prépare un composé de formule I dans lequel
X représente un atome de soufre,
R représente un atome de fluor ou de chlore, ou le groupe méthyle ou trifluorométhyle,
m est zéro, 1, 2 ou 3,
n est 1 ou, lorsque m est zéro, 1 ou 2, également 2,
R¹ représente un groupe alcényle en C₄-C₂₂, éventuellement un groupe CH₂ pouvant être remplacé par un atome d'oxygène,
R², R³, R⁴ représentent un atome d'hydrogène, de fluor ou de chlore, ou le groupe hydroxy, méthyle, méthoxy ou trifluorométhyle, R², R³, R⁴ étant identiques ou différents.

## Revendications (Revendications pour l'(les) Etat(s) contractant(s) suivant(s): GR)

1. Procédé pour la préparation de 3-thia- ou 3-oxa-alkylflavones substituées de formule I 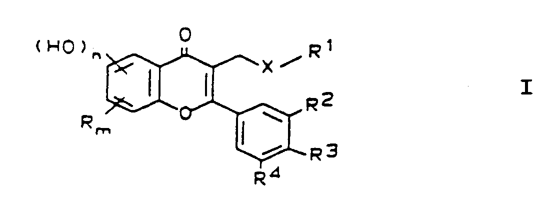 dans laquelle
X représente un atome de soufre ou d'oxygène,
R représente un atome d'halogène ou un groupe alkyle en C₁-C₄ ou trifluorométhyle;
m est zéro, 1, 2 ou 3,
n est 1 ou, lorsque m = zéro, 1 ou 2, également 2,
R¹ représente un radical alcényle en C₃-C₂₅, éventuellement un groupe CH₂ étant remplacé par un atome d'oxygène, ou un radical alcényle en C₃-C₂₅ qui est substitué par un groupe cyclohexényle qui, pour sa part, contient 1-3 groupes méthyle,
R², R³, R⁴ représentent un atome d'hydrogène ou d'halogène, ou un groupe hydroxy, alkyle en C₁-C₄, alcoxy en C₁-C₄, trifluorométhyle, R², R³, R⁴ étant identiques ou différents,
caractérisé en ce que
a) on convertit un phénol de formule II 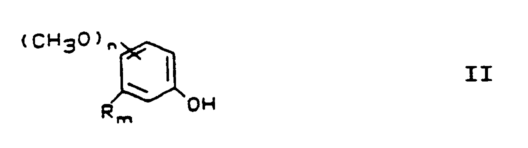 dans laquelle m, n et R ont les significations données à propos de la formule I,
par acylation, en les composés de formule III 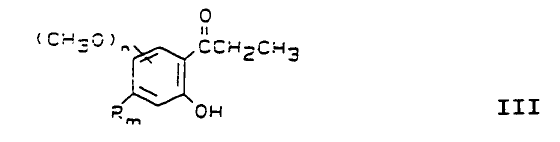 dans laquelle m, n et R ont les significations données à propos de la formule I,
b) on soumet un composé de formule III à une acylation sur le groupe hydroxy phénolique libre, avec un halogénure d'acide de formule IV 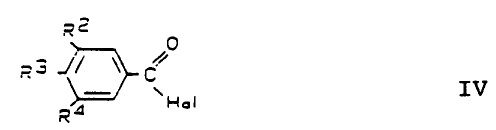 dans laquelle R², R³, R⁴ ont les significations données à propos de la formule I et Hal est un atome de chlore ou de brome,
pour aboutir à un composé de formule V 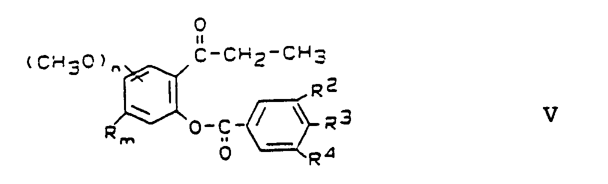 dans laquelle m, n, R et R², R³, R⁴ ont les significations données à propos de la formule I,
c) on cyclise un composé de formule V en un composé de formule VI 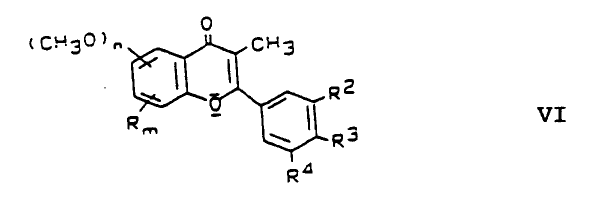 dans laquelle m, n, R et R², R³, R⁴ ont les significations données à propos de la formule I,
d) dans un composé de formule VI, on élimine les groupes méthoxy, pour aboutir à un composé de formule VII 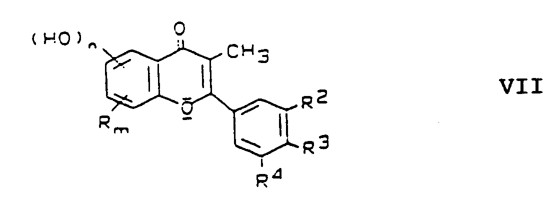 dans laquelle m, n et R, ainsi que R², R³, R⁴ ont les significations données à propos de la formule I,
e) on fait réagir un composé de formule VII avec un agent d'acylation, pour aboutir à un composé de formule VIII 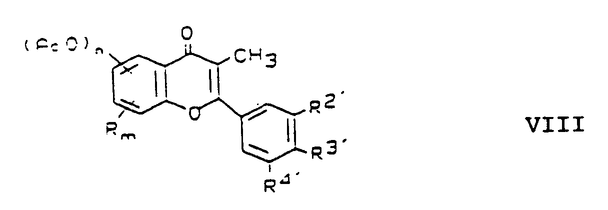 dans laquelle m, n et R ont les significations données à propos de la formule I, et R^{2'}, R^{3'}, R^{4'} représentent un atome d'hydrogène ou d'halogène, ou un groupe alkyle en C₁-C₄, alcoxy en C₁-C₄, trifluorométhyle ou acyloxy, R^{2'}, R^{3'}, R^{4'} étant identiques ou différents,
f) on soumet à une halogénation sélective un composé de formule VIII, pour aboutir à un composé de formule IX 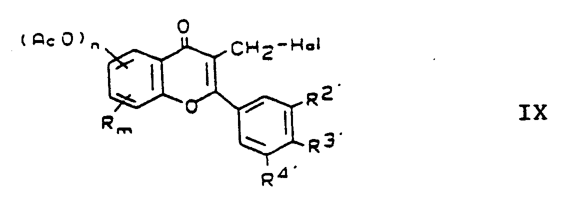 dans laquelle m, n et R ont les significations données à propos de la formule I, R^{2'}, R^{3'}, R^{4'} ont les significations données à propos de la formule VIII et Hal est un atome de chlore, brome ou iode,
g) on fait réagir un composé de formule IX avec un composé de formule X
H-X-R¹ X
dans laquelle X et R¹ ont les significations données à propos de la formule I,
pour aboutir à un composé de formule XI 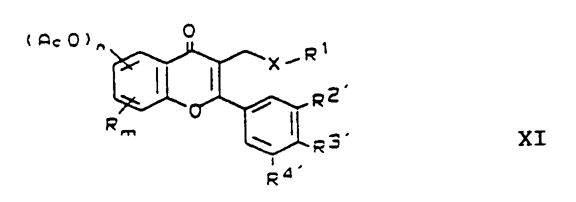 dans laquelle m, n, X, R et R¹ ont les significations données à propos de la formule I et R^{2'}, R^{3'}, R^{4'} ont les significations données à propos de la formule VIII,
h) dans un composé de formule générale XI, on élimine les groupes protecteurs acyle, pour aboutir à un composé de formule générale I.

2. Procédé pour la préparation d'une composition pharmaceutique, caractérisée en ce que l'on met sous une forme d'administration appropriée un composé préparable selon la revendication 1.

3. Composés de formule IX 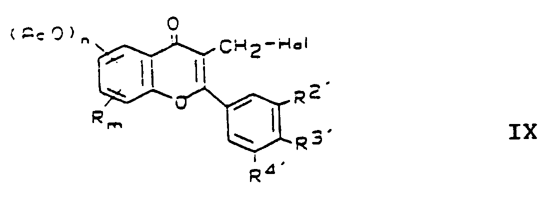 dans laquelle m, n et R ont les significations données à propos de la revendication 1 et R^{2'}, R^{3'}, R^{4'} ont les significations données à propos de la formule VIII dans la revendication 1 (e).

4. Composés de formule XI 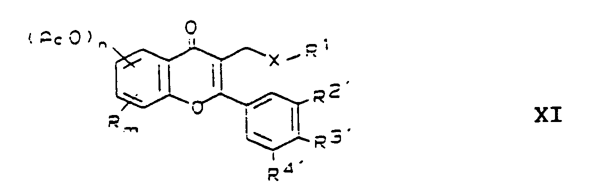 dans laquelle m, n, X, R et R¹ ont les significations données à propos de la formule I dans la revendication 1 et R^{2'}, R^{3'}, R^{4'} ont les significations données à propos de la formule VIII dans la revendication 1 (e).

5. Procédé selon la revendication 1, caractérisé en ce que l'on prépare un composé de formule I dans lequel,
X représente un atome de soufre ou d'oxygène,
R représente un atome de fluor ou de chlore, ou un groupe alkyle en C₁-C₃ ou trifluorométhyle,
m est zéro, 1, 2 ou 3,
n est 1 ou, lorsque m est zéro, 1 ou 2, également 2,
R¹ représente un groupe alcényle en C₄-C₂₂, éventuellement un groupe CH₂ pouvant être remplacé par un atome d'oxygène,
R², R³, R⁴ représentent un atome d'hydrogène, de fluor ou de chlore, ou un groupe hydroxy, alkyle en C₁-C₃, alcoxy en C₁-C₃ ou trifluorométhyle, R², R³, R⁴ étant identiques ou différents.

6. Procédé selon la revendication 1, caractérisé en ce que l'on prépare un composé de formule I dans lequel
X représente un atome de soufre,
R représente un atome de fluor ou de chlore, ou le groupe méthyle ou trifluorométhyle,
m est zéro, 1, 2 ou 3,
n est 1 ou, lorsque m est zéro, 1 ou 2, également 2,
R¹ représente un groupe alcényle en C₄-C₂₂, éventuellement un groupe CH₂ pouvant être remplacé par un atome d'oxygène,
R², R³, R⁴ représentent un atome d'hydrogène, de fluor ou de chlore, ou le groupe hydroxy, méthyle, méthoxy ou trifluorométhyle, R², R³, R⁴ étant identiques ou différents.
